# EUROPEAN PATENT APPLICATION

(11) **EP 4 671 761 A1**
(43) Date of publication of application: **31.12.2025**
(21) Application number: 24184039.6
(22) Date of filing: 24.06.2024
(51) Int. Cl.: G01N 33/543, G01N 33/558, G01N 33/68

(54) **USE OF HMGI-C FOR THE DETECTION OF ACUTE AORTIC SYNDROME**

(71) Applicant: PeriCard Check GmbH, 28757 Bremen (DE)
(72) Inventor: GLÜER, Salaheldien Ali Mohamed, 21149 Hamburg (DE)
(74) Representative: Peters, Hajo

(57) **Abstract**

The invention relates to the detection of HMGI-C, a protein (AAD-protein) released during an event leading to an acute aortic syndrome/acute aortic dissection (AAS/AAD) in a sample of bodily fluid of a human/patient. The invention thus provides a device for the detection of acute aortic dissection in a sample of bodily fluid of a mammal and thus of HMGI-C. This particularly includes a lateral flow device, the device comprising a strip, a sample pad for receiving the sample of the bodily fluid, a first conjugate pad with a conjugate comprising a protein antibody binding to HMGI-C, said AAD-protein, and a first detection band comprising a first conjugate-binding protein so that the presence of HMGI-C in the sample is indicated by a visible color change. In addition, the invention relates in other aspects also to a method for determination of the presence of AAD in a patient using an immunoassay as well as a more general method for the detection of acute aortic dissection in an individual by identifying HMGI-C in a sample of bodily fluid, especially in an individual by the use of an antibody binding to HMGI-C and thus identifying HMGI-C in a sample of bodily fluid.

## Description

### Field of the Invention

The invention relates to the detection of HMGI-C, a protein (AAD-protein) released during an event leading to an acute aortic syndrome/acute aortic dissection (AAS/AAD) in a sample of bodily fluid of a human/patient. The invention thus provides a device for the detection of acute aortic dissection in a sample of bodily fluid of a mammal and thus of HMGI-C. This particularly includes a lateral flow device, the device comprising a strip, a sample pad for receiving the sample of the bodily fluid, a first conjugate pad with a conjugate comprising a protein antibody binding to HMGI-C, said AAD-protein, and a first detection band comprising a first conjugate-binding protein so that the presence of HMGI-C in the sample is indicated by a visible color change. In addition, the invention relates in other aspects also to a method for determination of the presence of AAD in a patient using an immunoassay as well as a more general method for the detection of acute aortic dissection in an individual by identifying HMGI-C in a sample of bodily fluid, especially in an individual by the use of an antibody binding to HMGI-C and thus identifying HMGI-C in a sample of bodily fluid.

### Background of the Invention

Acute aortic dissection (AAD) is a life-threatening clinical entity requiring urgent surgical intervention. It is one aspect of acute aortic syndrome (AAS). The peak incidence of aortic dissection is reported to occur in the sixth and seventh decades of life with 2000 new cases per year in North America and 3000 in Europe, respectively.^{1,2} The initiating event is an intimal tear due to congenital defects of the aortic wall, such as in Marfan syndrome (MFS), Ehlers-Danlos syndrome (a heterogeneous group connective tissue disorders), idiopathic Erdheim Gsell medial necrosis, bicuspid aortic valve (the most common congenital heart disorder), or even acquired intima damage caused by hypertension, thoracic trauma or other unknown mechanism.³⁻⁵ Subintima or media necrosis results in intimal tear, which eventually allows blood to enter into the aortic wall, thus leading to separation of the aortic wall layers and hence to a rapid propagation of the aortic dissection. The separation of the media and the adventitia layers of the aortic wall may lead to malperfusion of numerous organs with devastating complications.^{2,6} An important clinical fact is that aortic dissections occur in both aneurysmatic expanded calibre as well as in normal calibre of the aorta. The most feared complication of aortic dissection is its outward rupture, which is associated with high mortality.⁷ The surgical treatment depends on the location of the initial tear and on the longitudinal propagation of the dissection. To describe dissections various classifications have been proposed, with the DeBakey and the Stanford classification more often used.^{8,9} When the ascending aorta is affected (Stanford type A), the mortality of untreated patients is about 36%-72% within the first 48 hours and 62%-91 % within the first week.¹⁰⁻¹³ Chest pain and other symptoms of AAD are often confused with those of cardiac infarction, thus complicating the diagnosis and delaying the treatment of AAD. Therefore, a better understanding of the molecular mechanisms underlying AAD can be the first step to supporting the future development of a rapid test for monitoring patients at high risk.

Given this fact it would be a huge benefit to provide ways that can suitably detect a marker, in the case of this invention HMGI-C, released during an event leading to an acute aortic syndrome/acute aortic dissection in samples from a human/patient, especially in a timely manner as time is all-important in this indication.

Accordingly, it would be particularly desirable to provide a lateral flow test. Lateral flow (LF) tests are membrane(strip)-based immunoassay tests. Lateral flow devices have a test strip consisting of a membrane, such as porous paper or a sintered polymer, which enables capillary flow of a sample and detection reagents. The membrane(strip) also holds substances that form test and control lines. One end of the membrane(strip) is fitted with a sample pad that contacts another pad used to store the detection conjugate. The other end of the membrane(strip) has a wicking pad that holds fluids and reagents that have migrated via capillary action through the membrane(strip). Lateral flow devices exist for a wide range of targets including the detection of infectious agents, metabolic molecules, antibodies, toxins, and drugs for use in human and veterinary applications.

Lateral flow tests are typically modelled on existing immunoassay formats and can be sandwich assays or competitive assays. Many assay variations are possible, but a positive signal is usually achieved by the specific accumulation of a detection complex at the test line.

Lateral flow devices usually need to be specifically developed in an iterative process for the desired application. Each component of the test strip of a lateral flow device requires specific evaluation followed by testing to determine the optimal combination of components. The identification of the most suitable membrane is an important step in the test strip development as this determines the capillary flow properties that need to be compatible with the type of sample to be tested.

A point-of-care lateral flow test that is rapid, reliable, and precise would be extremely useful in this regard and the current invention is exactly providing this.

As said above, the initiating event is an intimal tear due to defects of the aortic wall. The dissection of these layers occurs after the aortic rupture. After 5-10 min of the onset of pain, the biological marker HMGI-C (potentially also D-Dimer, aggrecan, smooth muscle myosin heavy chain, or soluble aortic elastin) is released in the blood. The biomarker level remains elevated for 6-10 days since the pain onset, thereby providing a greater one-time window for the detection of aortic wall damage. The high specificity of the biomarker measurements to the aortic wall damage was verified during surgical interventions, which consequently become a biomarker indicative of aortic dissection.

In addition, it could deliver a huge benefit if in these tests, proteins should be identified, whose blood (including Serum/Plasma) elevation/concentration differs between patients with aortic dissection and patients with myocardial infarction. Of note, MI and AAD often show the same symptoms, gold standard until today for MI diagnosis is based on "STE" on the 12-lead Electrocardiographic diagnostic.

It would be also particularly desirable to generally provide a device for the detection for the detection of acute aortic dissection in a sample of bodily fluid of a mammal and thus, preferably of HMGI-C/HMGA2.

It would also be particularly desirable to generally provide a method for the detection of acute aortic dissection in an individual by identifying HMGI-C/HMGA2 in a sample of bodily fluid.

It would further be particularly desirable to generally provide a method for the detection of acute aortic dissection in an individual by the use of an antibody binding to HMGI-C and thus identifying HMGI-C in a sample of bodily fluid.

Belge et al.¹⁵ describe the upregulation of the high mobility group AT-hook 2 gene in acute aortic dissection. Belge et al. do not consider/describe HMGA2 as a biomarker, especially in the blood, but instead analyzed tissue and expression levels.

CN 105 259 353 A¹⁶ describes the detection of ST2 as a biomarker in blood for AAD.

US 2013/0217015 A1¹⁷ describes HMGA2 as a biomarker for ovarian cancer.

### Summary of the Invention

In one aspect, the present invention provides a lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device further comprises a MI-protein antibody binding to a protein indicative for acute myocardial infarction (MI-protein) wherein the Ml-protein is troponin I.

In another aspect, the present invention provides a lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device is arranged so that if the sample reaches the first conjugate pad, the fluid in said first conjugate pad shows or turns to an alkaline pH.

In a further aspect, the present invention provides a lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device comprises an alkaline chemical buffer.

In another aspect the present invention provides a method for the immunoassay determination of acute aortic dissection (AAD) in a patient, said method comprising:
a) incubating a bodily fluid sample of the patient with
   a. at least one antibody to a first protein released during an event leading to an acute aortic dissection (1-AAD-protein) and
   b. a binding partner B for either 1-AAD-protein or the antibody

   wherein either the antibody or the binding partner B is conjugated to a detection agent,
   to form an immunological complex containing the detection agent;
b) isolating the immunological complex from the remaining sample; and
c) determining the detection agent either in the isolated complex or in the remaining sample as an indicator of the 1-AAD-protein in the sample;

thereby determining the occurrence of AAD in the patient,
wherein the 1-AAD-protein is HMGI-C; and
wherein step a) of incubating is done at an alkaline pH.

In a further aspect the present invention provides a method for the immunoassay determination of acute aortic dissection (AAD) in a patient, said method comprising:
a) incubating a bodily fluid sample of the patient with
   c. at least one antibody to a first protein released during an event leading to an acute aortic dissection (1-AAD-protein) and
   d. a binding partner B for either 1-AAD-protein or the antibody

   wherein either the antibody or the binding partner B is conjugated to a detection agent,
   to form an immunological complex containing the detection agent;
b) isolating the immunological complex from the remaining sample; and
c) determining the detection agent either in the isolated complex or in the remaining sample as an indicator of the 1-AAD-protein in the sample;
d) comparing the said value of said 1-AAD-protein with a known reference of the respective bodily fluid of a human without acute aortic dissection;

thereby determining the occurrence of AAD in the patient,
wherein the 1-AAD-protein is HMGI-C.

Another aspect of the present invention provides a method for the detection of acute aortic dissection (AAD) by detecting HMGI-C in a sample of bodily fluid of a subject, the method comprising:
1) contacting the sample with a protein antibody binding to HMGI-C and thus able to form HMGI-C/Antibody-conjugates;
2) detecting the presence and quantifying the amount of said HMGI-C/Antibody-conjugate in the sample following step 1); and
3) comparing the value obtained in step 3) with a known reference.
Preferably, the bodily fluid is blood, especially is full blood, blood plasma and/or blood serum.

### Brief Description of the Drawings

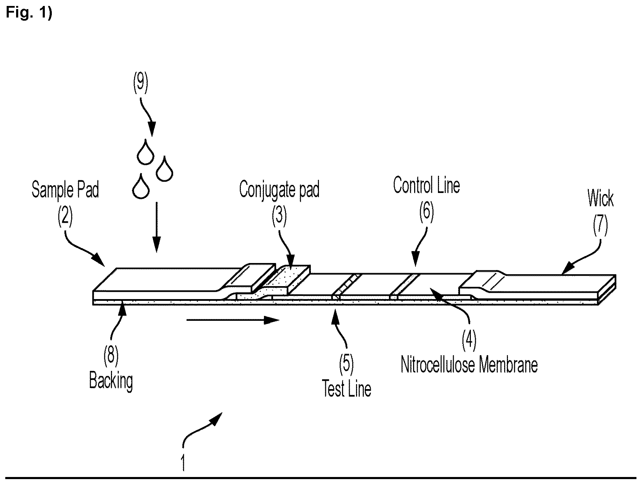

**Figure 1****)** is a schematic representation of a Lateral Flow Device According to the invention. The device (1) has a sample pad (2), a conjugate pad (3) including a mobilizable conjugate comprising an antibody protein binding to HMGI-C (said AAD-Protein) that has been conjugated to a detection agent, a membrane (4), and an optional wicking pad (7) for receiving and holding fluid that has travelled by capillary flow from the sample pad (2) and through the conjugate pad (3) and the membrane (4). A detection band (5) is shown which comprises an immobilized binding protein. Band (6) is an optional positive control band. The device (1) can further include a backing (8).

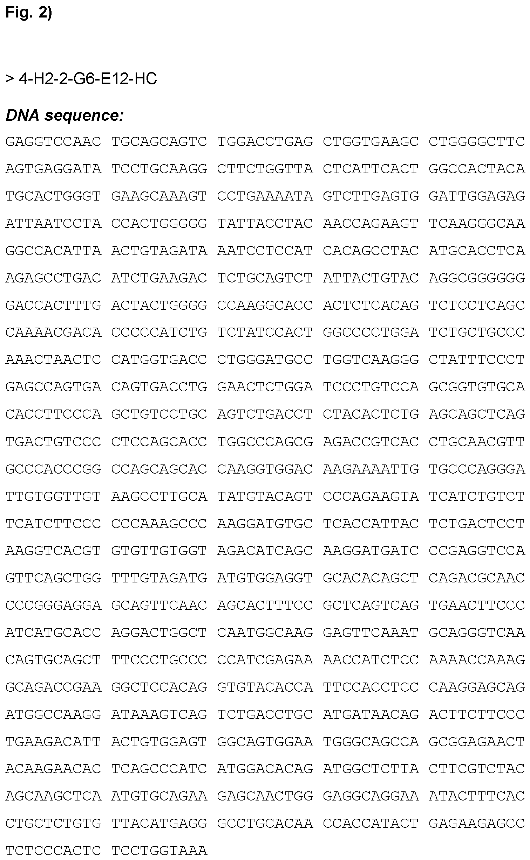

**Fig. 2****)** Nucleic acid sequence of the heavy chain variable domain (VH) of the antibody 4-H2 (SEQ ID NO. 5).

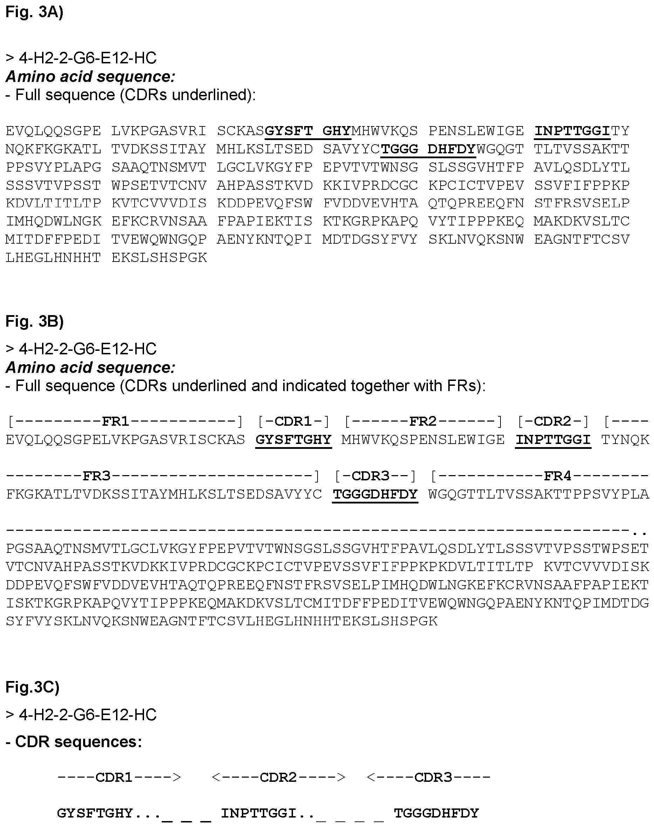

**Fig. 3A****)** Amino acid sequence of the heavy chain variable domain (VH) of the antibody 4-H2 (SEQ ID NO. 1). The CDRs of VH of 4-H2 are underlined and are as follow: CDR1 (SEQ ID NO. 2), CDR2 (SEQ ID NO. 3), and CDR3 (SEQ ID NO. 4).
**Fig. 3B****)** Amino acid sequence of the heavy chain variable domain (VH) of the antibody 4-H2 (SEQ ID NO. 1). The CDR1 (SEQ ID NO. 2), CDR2 (SEQ ID NO. 3) and CDR3 (SEQ ID NO. 4) in the chain are shown underlined, flanked by the framework regions, FR1 - FR4, as indicated.
**Fig. 3C****)** Amino acid sequence of the CDRs [CDR1 (SEQ ID NO. 2), CDR2 (SEQ ID NO. 3) and CDR3 (SEQ ID NO. 4)] of the heavy chain variable domain (VH) of the antibody 4-H2 (SEQ ID NO. 1).

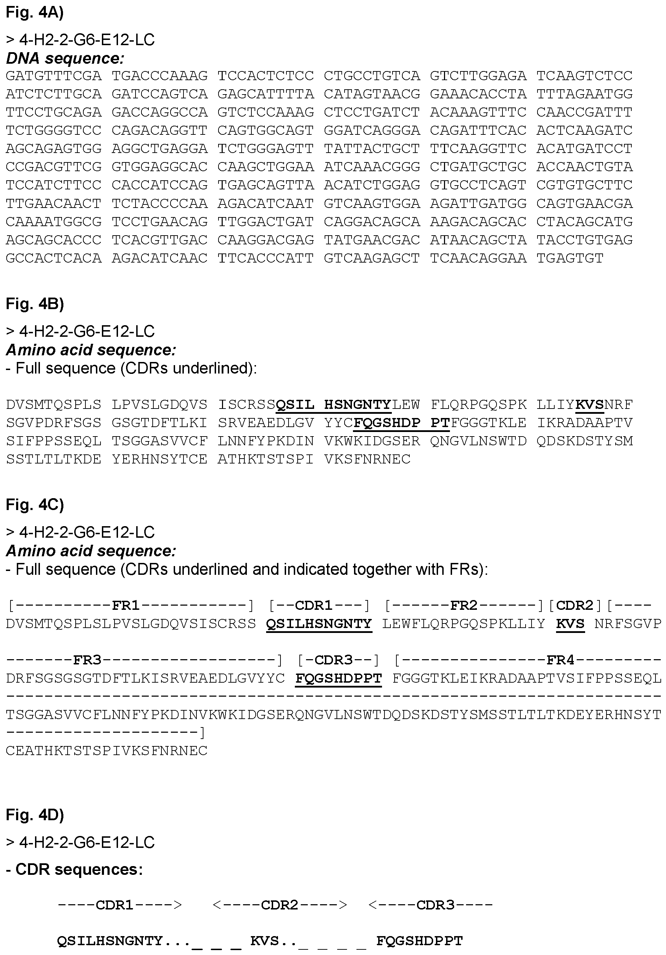

**Fig. 4A****)** Nucleic acid sequence of the light chain variable domain (VL) of the antibody 4-H2 (SEQ ID NO. 10).
**Fig. 4B****)** Amino acid sequence of the light chain variable domain (VL) of the antibody 4-H2 (SEQ ID NO. 6). The CDRs of VL of 4-H2 are underlined and are as follow: CDR1 (SEQ ID NO. 7), CDR2 (SEQ ID NO. 8), and CDR3 (SEQ ID NO. 9).
**Fig. 4C****)** Amino acid sequence of the light chain variable domain (VL) of the antibody 4-H2 (SEQ ID NO. 6). The CDR1 (SEQ ID NO. 7), CDR2 (SEQ ID NO. 8) and CDR3 (SEQ ID NO. 9) in the chain are shown underlined, flanked by the framework regions, FR1 - FR4, as indicated.
**Fig. 4D****)** Amino acid sequence of the CDRs [CDR1 (SEQ ID NO. 7), CDR2 (SEQ ID NO. 8) and CDR3 (SEQ ID NO. 9)] of the light chain variable domain (VL) of the antibody 4-H2 (SEQ ID NO. 1).

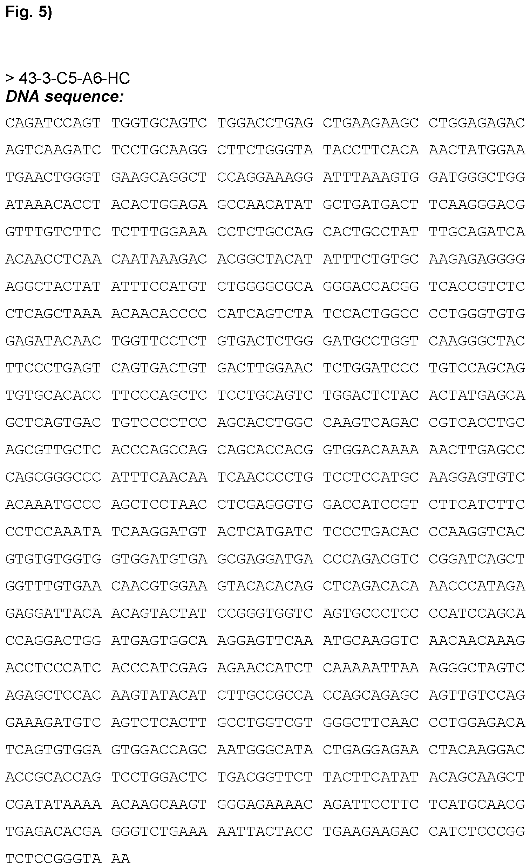

**Fig. 5****)** Nucleic acid sequence of the heavy chain variable domain (VH) of the antibody 43-3 (SEQ ID NO. 15).

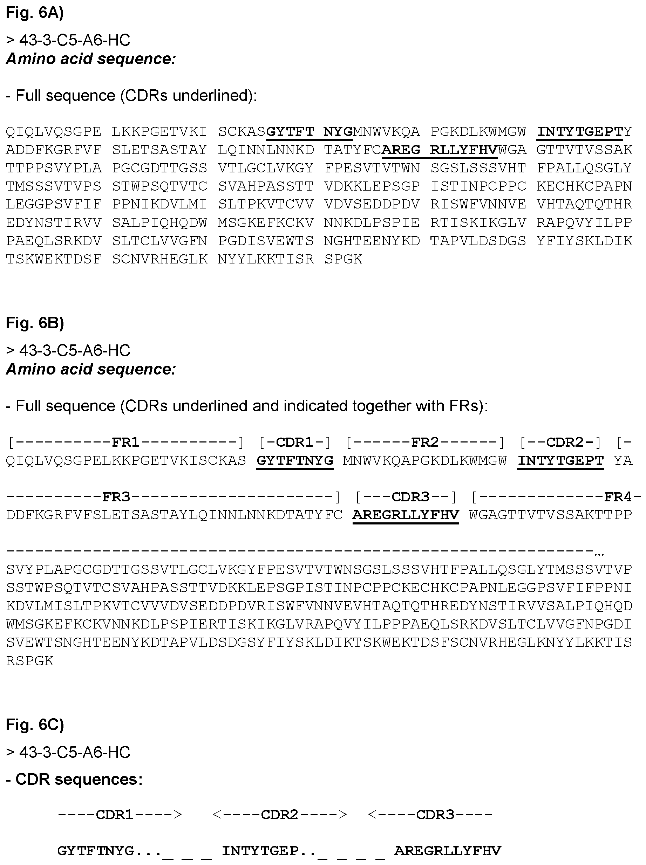

**Fig. 6A****)** Amino acid sequence of the heavy chain variable domain (VH) of the antibody 43-3 (SEQ ID NO. 11). The CDRs of VH of 43-3 are underlined and are as follow: CDR1 (SEQ ID NO. 12), CDR2 (SEQ ID NO. 13), and CDR3 (SEQ ID NO. 14).
**Fig. 6B****)** Amino acid sequence of the heavy chain variable domain (VH) of the antibody 43-3 (SEQ ID NO. 11). The CDR1 (SEQ ID NO. 12), CDR2 (SEQ ID NO. 13) and CDR3 (SEQ ID NO. 14) in the chain are shown underlined, flanked by the framework regions, FR1 - FR4, as indicated.
**Fig. 6C****)** Amino acid sequence of the CDRs [CDR1 (SEQ ID NO.2), CDR2 (SEQ ID NO.3) and CDR3 (SEQ ID NO. 14)] of the heavy chain variable domain (VH) of the antibody 43-3 (SEQ ID NO. 1).

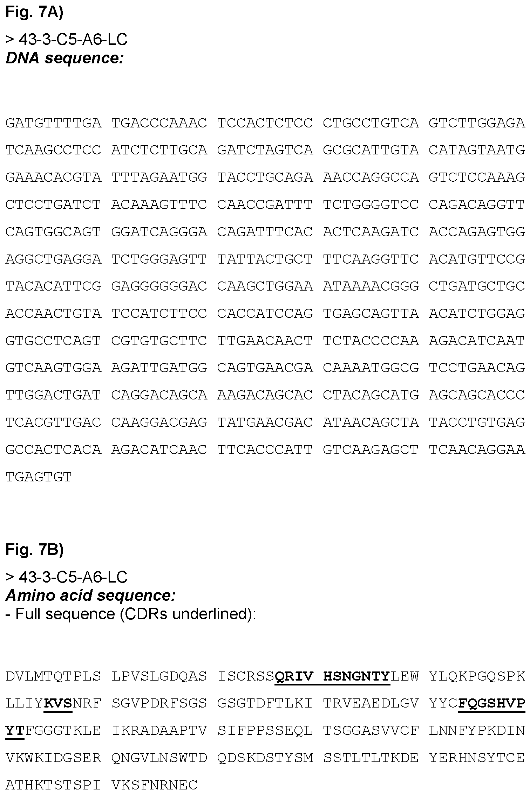

**Fig. 7A****)** Nucleic acid sequence of the light chain variable domain (VL) of the antibody 43-3 (SEQ ID NO. 20).
**Fig. 7B****)** Amino acid sequence of the light chain variable domain (VL) of the antibody 43-3 (SEQ ID NO. 16). The CDRs of VL of 43-3 are underlined and are as follow: CDR1 (SEQ ID NO. 17), CDR2 (SEQ ID NO. 18), and CDR3 (SEQ ID NO. 19).

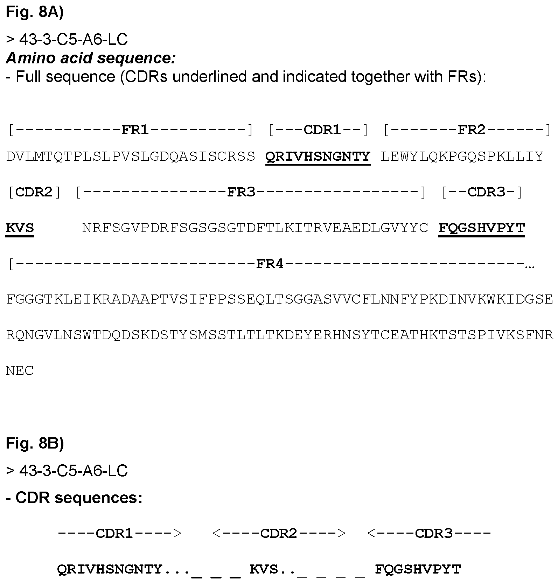

**Fig. 8A****)** Amino acid sequence of the light chain variable domain (VL) of the antibody 43-3 (SEQ ID NO. 16). The CDR1 (SEQ ID NO. 17), CDR2 (SEQ ID NO. 18) and CDR3 (SEQ ID NO. 19) in the chain are shown underlined, flanked by the framework regions, FR1 - FR4, as indicated.
**Fig. 8B****)** Amino acid sequence of the CDRs [CDR1 (SEQ ID NO. 17), CDR2 (SEQ ID NO. 18) and CDR3 (SEQ ID NO. 19)] of the light chain variable domain (VL) of the antibody 43-3 (SEQ ID NO. 1).

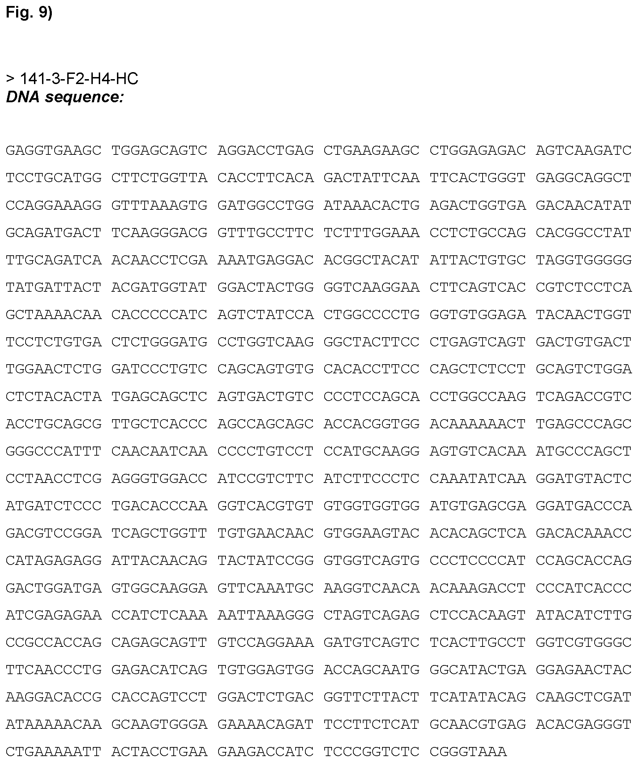

**Fig. 9****)** Nucleic acid sequence of the heavy chain variable domain (VH) of the antibody 141-3 (SEQ ID NO. 15).

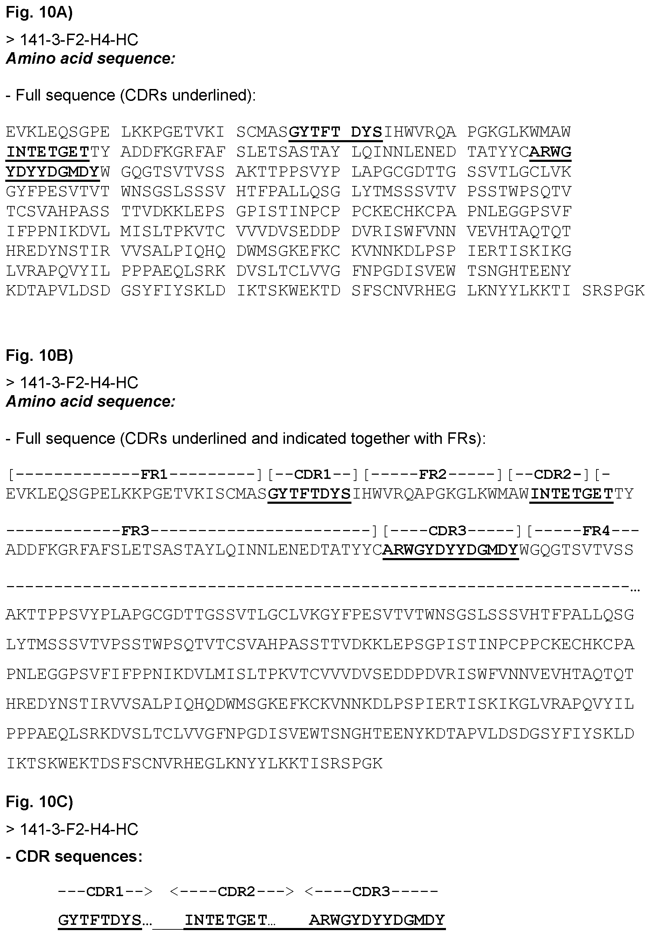

**Fig. 10A****)** Amino acid sequence of the heavy chain variable domain (VH) of the antibody 141-3 (SEQ ID NO. 11). The CDRs of VH of 43-3 are underlined and are as follow: CDR1 (SEQ ID NO. 12), CDR2 (SEQ ID NO. 13), and CDR3 (SEQ ID NO. 14).
**Fig. 10B****)** Amino acid sequence of the heavy chain variable domain (VH) of the antibody 141-3 (SEQ ID NO. 11). The CDR1 (SEQ ID NO. 12), CDR2 (SEQ ID NO. 13) and CDR3 (SEQ ID NO. 14) in the chain are shown underlined, flanked by the framework regions, FR1 - FR4, as indicated.
**Fig. 10C****)** Amino acid sequence of the CDRs [CDR1 (SEQ ID NO.2), CDR2 (SEQ ID NO.3) and CDR3 (SEQ ID NO. 14)] of the heavy chain variable domain (VH) of the antibody 141-3 (SEQ ID NO. 1).

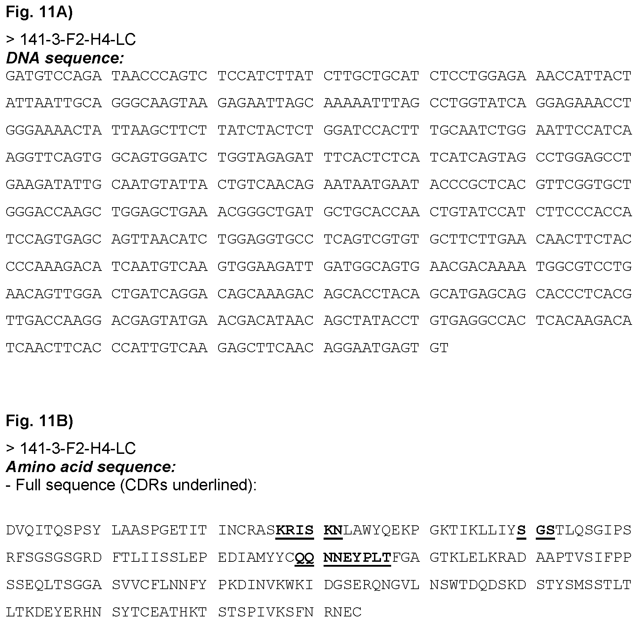

**Fig. 11A****)** Nucleic acid sequence of the light chain variable domain (VL) of the antibody 141-3 (SEQ ID NO. 20).
**Fig. 11B****)** Amino acid sequence of the light chain variable domain (VL) of the antibody 141-3 (SEQ ID NO. 16). The CDRs of VL of 43-3 are underlined and are as follow: CDR1 (SEQ ID NO. 17), CDR2 (SEQ ID NO. 18), and CDR3 (SEQ ID NO. 19).

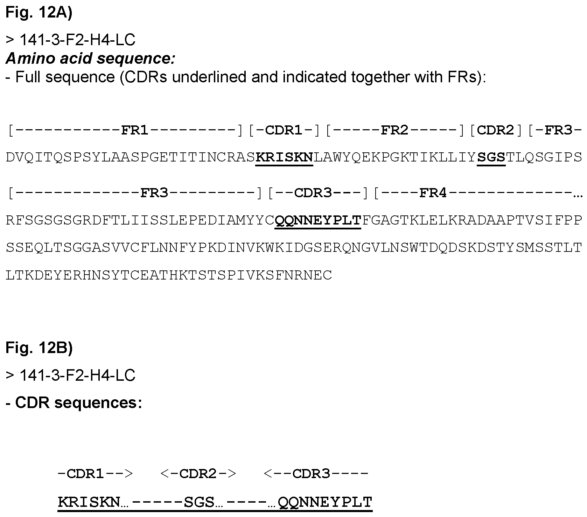

**Fig. 12A****)** Amino acid sequence of the light chain variable domain (VL) of the antibody 141-3 (SEQ ID NO. 16). The CDR1 (SEQ ID NO. 17), CDR2 (SEQ ID NO. 18) and CDR3 (SEQ ID NO. 19) in the chain are shown underlined, flanked by the framework regions, FR1 - FR4, as indicated.
**Fig. 12B****)** Amino acid sequence of the CDRs [CDR1 (SEQ ID NO. 17), CDR2 (SEQ ID NO. 18) and CDR3 (SEQ ID NO. 19)] of the light chain variable domain (VL) of the antibody 141-3 (SEQ ID NO. 1).

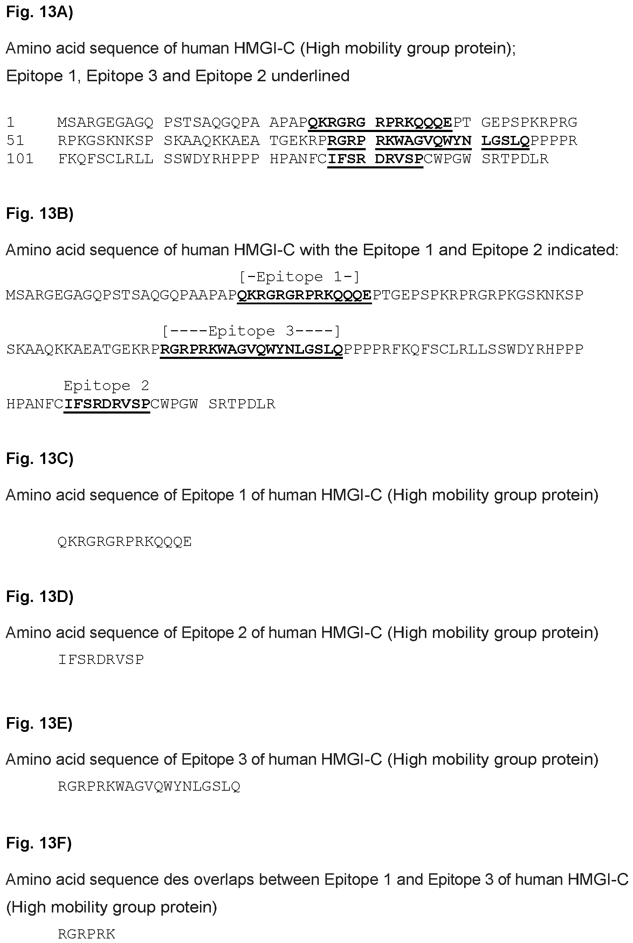

**Fig. 13A****)** Amino acid sequence of human HMGI-C (High mobility group protein)
**Fig. 13B****)** Amino acid sequence of human HMGI-C (SEQ ID NO. 31) with Epitope 1 and Epitope 2 shown and marked.
**Fig. 13C****)** Amino acid sequence of an epitope (designated Epitope 1) of human HMGI-C (SEQ ID NO. 32).
**Fig. 13D****)** Amino acid sequence of an epitope (designated Epitope 2) of human HMGI-C (SEQ ID NO. 33).
**Fig. 13E****)** Amino acid sequence of an epitope (designated Epitope 3) of human HMGI-C (SEQ ID NO. 34).
**Fig. 13F****)** Amino acid sequence of the overlap between Epitope 1 and Epitope 3 of human HMGI-C (SEQ ID NO. 35).
**Fig. 14A****)** This figure shows an SDS PAGE and shows the purified antibody production from the final clones 43-3-C5-A6 and 4-H2-2-G6-E12 of Example 1 with
   - lane no. 1 showing antibody-43-3-C5-A6 (PCC-2) (2µg, 10% reduce SDS-PAGE)
   - lane no. 2 showing antibody-4-H2-2-G6-E12 (PCC-1) (2µg, 10% reduce SDS-PAGE)
   - lane no. 3 showing antibody-43-3-C5-A6 (PCC-2) (2µg, 10% non-reduce SDS-PAGE)
   - lane no. 4 showing antibody-4-H2-2-G6-E12 (PCC-1) (2µg, 10% non-reduce SDS-PAGE).

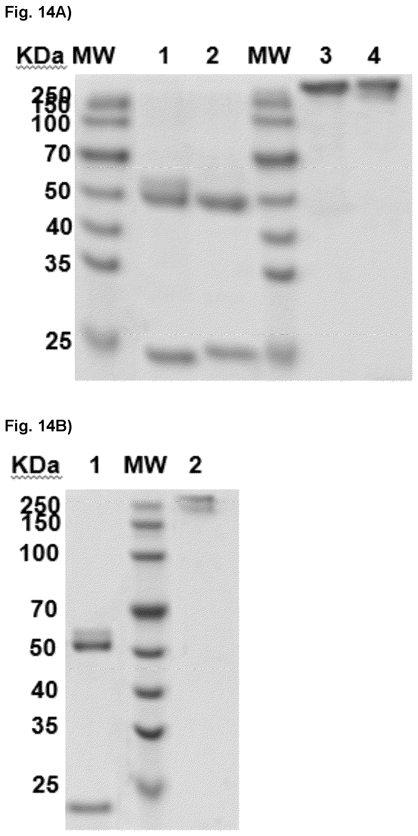

**Fig. 14B****)** This figure shows an SDS PAGE and shows the purified antibody production from the final clone 141-3-F2-H4 (PCC-3) of Example 1 with
   - lane no. 1 showing antibody-141-3-F2-H4 (PCC-3) (2µg, 10% reduce SDS-PAGE)
   - lane no. 2 showing antibody-141-3-F2-H4 (PCC-3) (2µg, 10% non-reduce SDS-PAGE)

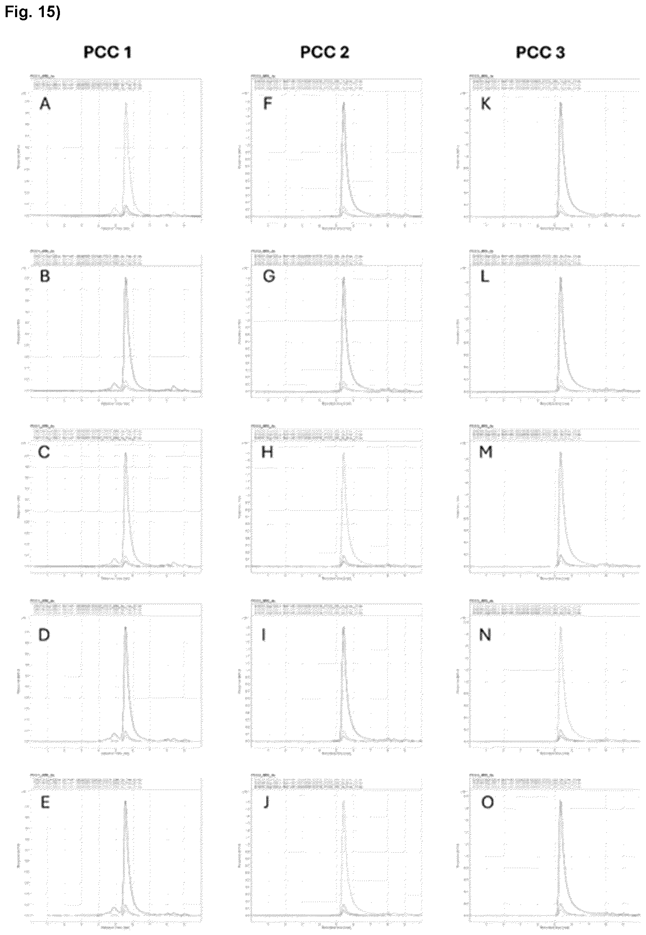

**Fig. 15****)** This figure shows a HPLC analysis of Antibody "43-3" (PCC-2), Antibody "4-H2" (PCC-1) and Antibody "141-3" (PCC-3) as described in Example 3. (A-E) 17 µg/ml of the Antibody PCC1, (F-J) PCC2 and (K-O) PCC3 were analyzed by HPLC. For the analyses by HPLC samples were diluted in NaPO4 Buffer 150 mM pH 7. Antibodies were analyzed at wavelength 220.4 nm, 280,4 nm and 158,4 nm. n = 5 technical replicates

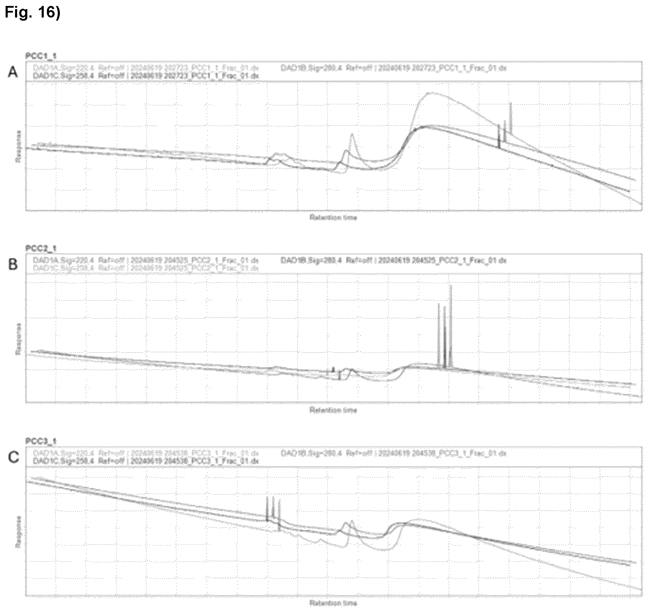

**Fig. 16****)** This figure shows a further HPLC analysis of Antibody "43-3" (PCC-2), Antibody "4-H2" (PCC-1) and Antibody "141-3" (PCC-3) as described in Example 4.
   HPLC analyses of the denatured antibodies PCC1, PCC2 and PCC3. (A) 25 µg/ml of the Antibody PCC1, (B) PCC2 and (C) PCC3 were analyzed by HPLC. For the analyses by HPLC samples were diluted in NaPO₄ Buffer 150 mM pH 7. Antibodies were analyzed at wavelength 220.4 nm, 280,4 nm and 158,4 nm.

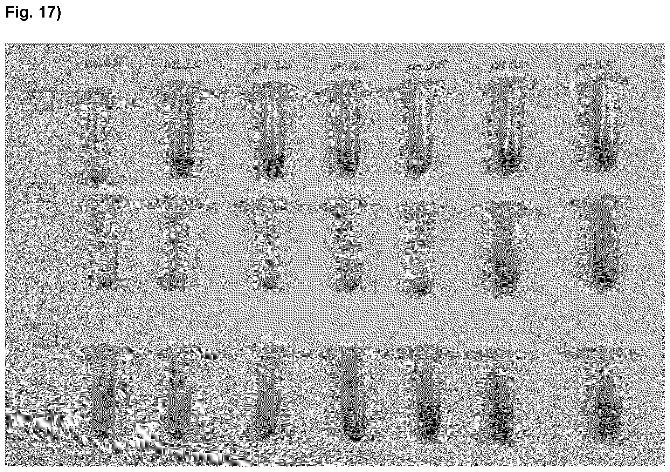

**Fig. 17****)** This figure shows pH measurements of the respective antibodies Antibody "43-3" (PCC-2), Antibody "4-H2" (PCC-1) and Antibody "141-3" (PCC-3) if conjugated as described in Example 5.

Other objects, aspects, features and advantages of the present invention will become apparent from the following description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### Detailed Description of the invention

### Definitions

Throughout this specification, unless the context requires otherwise, the words "comprise," "comprises," and "comprising" will be understood to imply the inclusion of a stated step or element or group of steps or elements but not the exclusion of any other step or element or group of steps or elements. It should also be understood that a term with the words "comprise," "comprises," and "comprising" can be in a limiting way be replaced to mean the words "consists of', "consists of" and "consisting of", and then be understood to imply the inclusion of a stated step or element or group of steps or elements but the exclusion of any other step or element or group of steps or elements.

The terms "protein", "protein fragment", "polypeptide", and "peptide" may be used interchangeably herein to refer to a polymer of amino acid residues and to variants and synthetic and naturally occurring analogues of the same. Thus, these terms apply to amino acid polymers in which one or more amino acid residues are synthetic non-naturally occurring amino acids, such as a chemical analogue of a corresponding naturally occurring amino acid, as well as to naturally- occurring amino acid polymers and naturally occurring chemical derivatives thereof.

The term "antibody" or "protein antibody", also referred to as "immunoglobulin", is a Y-shaped protein of the immune system that specifically identifies foreign objects or antigens, such as the components of bacteria, yeasts, parasites, and viruses. Each tip of the 'Y' of an antibody contains an antigen-binding site that is specific for a particular epitope on an antigen, allowing these two structures to bind together with precision. Thus, an "antibody" typically comprises two light and two heavy chains. An "antibody" typically comprises all or a portion of an Fc region, to facilitate detection by an Fe-binding protein, and may also comprise one or more antigen-binding sites, to facilitate detection by an antibody-specific binding agent, such as an antigen or antigenic peptide. The antibodies can be, e.g., of IgG, IgE, IgD, IgM, or IgA type.

The term "antigen" means a molecule having distinct surface features or epitopes capable of stimulating a specific immune response. Antibodies (immunoglobulins) are produced by the immune system in response to exposure to antigens. Antigens maybe proteins, carbohydrates or lipids, although only protein antigens are classified as immunogens because carbohydrates and lipids cannot elicit an immune response on their own.

An "antigen-binding site," or "binding portion" of an antibody, refers to the part of the immunoglobulin molecule that participates in antigen binding. The antigen-binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". In an antibody molecule, the three hypervariable regions of a light chain and the three hypervariable regions of a heavy chain are disposed relative to each other in three dimensional space to form an antigen-binding surface. The antigen-binding surface is complementary to the three dimensional surface of a bound antigen, and the three hypervariable regions of each of the heavy and light chains are referred to as "complementarity-determining regions," or "CDRs."

The term "Fc-binding protein" refers to a protein or polypeptide capable of binding to the fragment crystallizable region (Fc region) of an antibody. The Fc region is the tail region of an antibody that interacts with cell surface Fc receptors and certain proteins of the complement system. In IgG, IgA and IgD antibody isotypes, the Fc region is composed of two identical protein fragments, derived from the second and third constant domains of the antibody's two heavy chains. IgM and IgE Fc regions contain three heavy chain constant domains (CH domains 2-4) in each polypeptide chain. The Fc regions of IgG antibodies bears a highly conserved N-glycosylation site. The N-glycans attached to this site are predominantly core-fucosylated diantennary structures of the complex type. In addition, small amounts of these N-glycans also bear bisecting GlcNAc and. alpha-2,6 linked sialic acid residues. The Fab region of an antibody contains variable sections that define the target-specificity of the antibody, and in contrast, the Fc region of all antibodies in a class are the same for each species; they are constant rather than variable.

The term "nanoparticles" means uniform particles having a size of 1-200 nm.

The term "nanoshells" means nanoparticles that consist of a core and a metallic shell (usually gold).

In the context of this invention the term "acute aortic dissection (AAD)" is to be understood as a clinical entity being part of the "AAS", initiated by an intimal tear due to congenital defects of the aortic wall, such as in Marfan syndrome (MFS), Ehlers-Danlos syndrome (a heterogeneous group connective tissue disorders), idiopathic Erdheim Gsell medial necrosis, bicuspid aortic valve (the most common congenital heart dissection), or even acquired intima damage caused by hypertension, thoracic trauma or other unknown mechanism.³⁻⁵ One complication of aortic dissection is its outward rupture, which is surgically treated. With the Stanford type A the mortality of untreated patients is about 36%-72% within the first 48 hours and 62%-91 % within the first week.¹⁰⁻¹³.

In the context of this invention the term "acute aortic syndrome (AAS)" is to be understood as a broader term including the AAD. The AAS (as well as the AAD) can include the following syndroms: Penetrating Aortic Ulcer and Intramural Hematoma, Thoracic Aortic Aneurysm, Chronic Dissection-Indications with Treatment with Branched and Fenestrated Stent-grafts, Different Therapeutic Modalities for Aortic Arch Dissection Combined with Kommerell's Diverticulum, Secondary Interventions After Endovascular Repair of Aortic Dissections, False Aneurysms Complicating Internal Carotid Artery Dissection, Established Indications for the Invasive Treatment of a Thoracic Aortic Aneurysm, Aortic Arch Dissection: A Controversy of Classification, Treatment Algorithms for Patients with (Sub)acute Type B Aortic Dissections, Descending Thoracic Aortic Dissections, Differences in Aortopathy in Patients with a Bicuspid Aortic Valve with or Without Aortic Coarctation, Angina Chest pain, Dyspnea Accompanied by Hypertension Crisis, Sudden Coma or Shock of Unknown Etiology.

In the context of this invention the term "1-AAD-protein" is HMGI-C released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "1-AAD-protein" is released in the blood. The biomarker ("1-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection.

In the context of this invention the term "2-AAD-protein" is to be understood as one or more from a group of proteins of which one or more are released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "2-AAD-protein/s" is/are released in the blood. The biomarker ("2-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection. The "2-AAD-proteins" can be D-Dimer but it could also be aggrecan, smooth muscle myosin heavy chain, or soluble aortic elastin.

In the context of this invention the term "X-AAD-protein" is to be understood as one or more from a group of proteins of which one or more are released into the blood after the initiating event of the acute aortic syndrome/acute aortic dissection (AAS/AAD). This initiating event is an intimal tear due to defects of the aortic wall. The dissection of these layers occurs after the aortic rupture. After 5-10 min of the onset of pain, one or more of these biological markers, the "X-AAD-protein/s" is/are released in the blood. The biomarker ("X-AAD-protein") level remains elevated for 6-10 days since the pain onset, thereby providing a great one-time window for the detection of aortic wall damage. The high specificity of the biomarker measurements to the aortic wall damage was verified during surgical interventions, which consequently become a biomarker indicative of aortic dissection. The "X-AAD-proteins" can be e.g. D-dimer, aggrecan, smooth muscle myosin heavy chain, or soluble aortic elastin.

In the context of this invention the term "biomarker" is to be understood as any compound of any biological origin whose detection signals the occurrence of a event in an organism. In the context of this invention "biomarker" is especially understood as one or more proteins, especially enzymes, whose presence in a bio-sample for acute aortic dissection (AAD)more especially the above-described "1-AAD-protein/s", "2-AAD-protein/s" or "X-AAD-protein/s".

In the context of this invention the term "sample pad" is to be understood as a stretch on (or a part of) the strip onto which a fluid sample or analyte, preferably a sample of body fluids, especially of blood, e.g. of blood from a human/ a patient is applied. This sample pad can e.g. comprise or consist of polymers or paper or cotton, like sintered polymer, cotton, porous paper or a microstructured polymer or nitrocellulose. The sample pad should be constructed so that the bodily fluid can be - e.g. by way of an opening in the lateral flow device - applied to it. The sample pad can also act as a sponge and is often able to hold excess fluid.

In the context of this invention the term "bodily fluid" is to be understood as any fluid inside a body, especially that of a mammal, like a human or a patient. A body fluid or bodily fluid can be blood, urine, semen, sputum, saliva etc., most preferably is blood like whole blood, blood serum or blood plasma etc.

In the context of this invention the term "strip" is to be understood as a material or combination of materials enabling capillary flow of fluid along - at least - a portion of the strip. The strip may have - and preferably has - the capacity to spontaneously transport the fluid, like bodily fluid, like blood. The strip can comprise or consist of polymers or paper, like sintered polymer, a microstructured polymer, nitrocellulose, porous paper, especially porous paper like nitrocellulose.

In the context of this invention the term "conjugate" is to be understood as a protein antibody binding to a biomarker (see above) that has been conjugated to a detection agent. In a preferred embodiment said protein antibody is an antibody that binds to a 1-AAD-protein, 2-AAD-protein or X-AAD-protein (see above) and which is conjugated to a detection agent (a Tag) like gold, colored latex etc. Often, the antibody is freeze-dried and/or is a monoclonal antibody. Preferably, regarding 1-AAD-protein, the antibody in the conjugate is an antibody binding to HMGI-C. Preferably in the conjugate this antibody is conjugated to a gold-particle (colloidal gold) or a colored latex-particle, more preferably a (colloidal) gold particle. Most preferably , regarding 1-AAD-protein, the conjugate is a monoclonal antibody binding to HMGI-C conjugated with a gold-particle.

In the context of this invention the term "conjugate pad" is to be understood as a stretch on (or a part of) the strip into which the fluid sample or analyte, previously applied to the sample pad, is transported via capillary flow, e.g. in the strip. It does contain (comprise) a conjugate (see above). This conjugate pad can e.g. consist of polymers or paper, like sintered polymer, porous paper or a microstructured polymer or nitrocellulose. When the sample or analyte flows under capillary action through the strip from the sample pad to the conjugate pad, it mobilizes the conjugate contained (comprised) in the conjugate pad.

In the context of this invention the term "mobilize" is to be understood as being displaced from its current position, e.g. inside the conjugate pad. Preferably, this is applied to the movement of the conjugate (see above) contained inside the conjugate pad further along the strip along with the sample/fluid, e.g. by capillary forces, e.g. towards/to the "detection band". Most preferably, this is applied to the movement of the conjugate (see above) contained inside the conjugate pad which is bound through its protein antibody to the biomarker (1-AAD-protein, 2-AAD-protein, or X-AAD-protein) from the sample/analyte further along the strip along with the sample/fluid.

In the context of this invention "the 1-AAD-protein antibody conjugate" (or the "2-AAD-protein antibody conjugate") is the conjugate (see above) of the "protein antibody binding to" the "biomarker", 1-AAD-protein (or 2-AAD-protein), with a "detection agent". This also covers the conjugate (see above) of the "protein antibody binding to" the "biomarker", 1-AAD-protein (or 2-AAD-protein), with a "detection agent" being bound through its protein antibody to the biomarker 1-AAD-protein (or 2-AAD-protein).

In the context of this invention "the mobilized 1-AAD-protein antibody conjugate" (or the mobilized 2-AAD-protein antibody conjugate) is the conjugate (see above) of the "protein antibody binding to" the "biomarker", 1-AAD-protein (or 2-AAD-protein), with a "detection agent", being mobilized e.g. from the conjugate pad and moved along with the sample. This also covers the conjugate (see above) of the "protein antibody binding to" the "biomarker", 1-AAD-protein (or 2-AAD-protein), with a "detection agent" being bound through its protein antibody to the biomarker 1-AAD-protein (or 2-AAD-protein), being mobilized e.g. from the conjugate pad and moved along with the sample.

In the context of this invention the term "protein antibody" is to be understood as an antibody being a protein that binds to a biomarker (see above). In a preferred embodiment said protein antibody is an antibody that binds to a 1-AAD-protein, 2-AAD-protein or X-AAD-protein (see above). Often, the antibody is freeze-dried and/or is a monoclonal antibody. Preferably, regarding 1-AAD-protein, the protein antibody is an antibody binding to HMGI-C, more preferably is a monoclonal antibody binding to HMGI-C. It can also be preferred, regarding s-AAD-protein or X-AAD-protein, that the protein antibody is an antibody binding to D-Dimer, more preferably is a monoclonal antibody binding to D-Dimer.

In the context of this invention the term "protein antibody binding to" is to be understood as a protein antibody wherein it is clarified to which antigen it binds. Usually the "protein antibody binding to" binds to a biomarker (see above) and in a preferred embodiment said protein antibody binds to a 1-AAD-protein, 2-AAD-protein or X-AAD-protein (see above) as its antigen. Preferably, regarding 1-AAD-protein, it is a protein antibody binding to HMGI-C, more preferably is a monoclonal antibody binding to HMGI-C.

In the context of this invention the term "detection agent" is to be understood as a tag like gold, colored latex, or gold and colored latex-particles or magnetic beads. Preferably the detection agent is understood as part of the conjugate described above, being preferably a gold or latex particle being conjugated to the protein antibody (see above).

In the context of this invention the term "detection band" is to be understood as a stretch on the strip into which the fluid sample or analyte together with the mobilized conjugate (which comprises the "protein antibody"), contained in the conjugate pad, is transported via capillary flow, e.g. in the strip. The detection band is located substantially perpendicular to the direction of flow of the sample along the strip. The detection band does comprise (contain) a (first or second) conjugate-binding protein (see below). The (first or second) conjugate-binding protein is immobilized within the strip (the detection band), preferably along a band (the detection band) located substantially perpendicular to the direction of flow of the sample along the strip. Thus, when the flow of the sample together with the mobilized (1- or 2-)AAD-protein antibody conjugate in the sample reaches the detection band, it contacts (or - at least some AAD-protein antibody conjugate - binds to) the first conjugate-binding protein comprised (contained) within the detection band. This leads to that the presence of said (1- or 2-)AAD-protein in the sample is indicated by a visible color change. This detection band can e.g. consist of polymers or paper, like sintered polymer, porous paper or a microstructured polymer or nitrocellulose.

In the context of this invention the term "immobilized" is to be understood as first of all meaning that it is comprised/contained within the detection band and within the strip. Preferably, it means that it is not mobilized when fluid contacts the immobilized entity, even if continues to flow by capillary flow. Thus, it means that it (e.g. the immobilized (first or second) conjugate-binding protein) is not mobilized by the fluid sample if it (together with the mobilized (1- or 2-)AAD-protein antibody conjugate in the sample) reaches (and contacts) the "immobilized" (first or second) conjugate-binding protein in the detection band.

In the context of this invention the term "contact" or "contacts" is to be understood as one entity getting in contact with another entity. It also includes "binding" or "binds". Thus, "contacts" also includes if a "conjugate-binding-protein" binds to a (mobilized) "AAD-protein antibody conjugate" like a "mobilized 1-AAD-protein antibody conjugate".

In the context of this invention the term "conjugate binding site" is to be understood as covering two different embodiments: a) the "conjugate-binding protein" (as described directly below) or b) a construct in which a surface-structure (site) of the biomarker, the 1-AAD-protein, 2-AAD-Protein or X-AAD-protein is presented to the fluid, e.g. of the sample. In this, the conjugate binding site is immobilized within the strip, usually at a detection band, usually the first detection band, but it could also be immobilized in another (2^{nd}) detection band. In that, in a sample containing the mobilized 1-AAD-protein antibody conjugate (the mobilized 2-AAD-protein antibody conjugate), the mobilized 1-AAD-protein antibody conjugate (the mobilized 2-AAD-protein antibody conjugate) could bind to the conjugate binding site if the "protein antibody" is not already bound to the biomarker/AAD-protein.

In the context of this invention the term "conjugate-binding protein" is to be understood as a protein that is binding to a conjugate comprising an antibody. Preferably, the conjugate-binding protein is binding to the "protein antibody" (described above). The "conjugate-binding protein" binds a) either directly to the "protein antibody" (described above) being part of the "conjugate" (descirbed above) or b) indirectly to the "protein antibody" (described above) by binding to the biomarker as an antigen (1-AAD-protein, 2-AAD-protein, or X-AAD-protein) which is bound via the "protein antibody" of the "conjugate" (described above). Most importantly, though, the "conjugate-binding protein" is - highly preferably - only binding to the protein antibody of the conjugate - whether directly or indirectly - if the protein antibody of the conjugate is - preferably already - bound to the biomarker/AAD-protein. This would be the case in a preferred embodiment of the invention in which the LFA is executed as a Sandwich assay. It is to be understood that the term "contact" also includes "binding" or "binds" in the context of "conjugate-binding protein". The "conjugate-binding protein" is immobilized within the strip, usually at a detection band, usually the first detection band, but it could also be immobilized in another (2^{nd}) detection band. In a preferred embodiment, the "conjugate-binding protein" is directly binding to a biomarker as antigen, which is also bound to the protein antibody of the conjugate; the conjugate being mobilized from the conjugate pad by the fluid from the sample with the protein antibody of the conjugate bound to the biomarker. In a very preferred embodiment, regarding 1-AAD-protein, the "conjugate-binding protein" is directly binding to HMGI-C, which is also bound to the protein antibody of the conjugate; the conjugate being mobilized from the conjugate pad by the fluid from the sample with the protein antibody of the conjugate bound to HMGI-C. As an alternative the "conjugate-binding protein" is an FC-binding protein, preferably wherein the FC-binding protein is Protein G, Protein A or a Protein A/G fusion protein.

If the "conjugate-binding protein" binds to the same biomarker as the "protein antibody" (in the "conjugate"), the "conjugate-binding protein" can bind to the same epitope on the same biomarker as the "protein antibody" (in the "conjugate") or the "conjugate-binding protein" can bind to a different epitope on the same biomarker than the "protein antibody" (in the "conjugate").

In the context of this invention the term "further conjugate-binding protein" is to be understood as a protein that is binding to a mobilized MI-protein antibody sample conjugate comprising an antibody to an MI-protein (see below). The further conjugate-binding protein" is different from the first and second immobilized conjugate-binding protein. The "further conjugate-binding protein" binds a) either directly to the "MI-protein antibody" (described below) being part of a conjugate or b) indirectly to the "MI-protein antibody" (described below) by binding to the Ml-protein, 2-AAD-protein, or X-AAD-protein) which is bound via the "MI-protein antibody" being conjugated. The "further conjugate-binding protein" is only binding to the MI-protein antibody being conjugated - whether directly or indirectly - if the MI-protein antibody being conjugated is bound to the MI-protein.

In the context of this invention the term "the presence or absence of color" is to be understood as either a) a "visible color change" of the detection band or b) no change of color in the detection band when the mobilized (1-; or 2-)AAD-protein antibody conjugate in the sample contacts the (first or second) conjugate-binding protein in the (first or second) detection band.

In the context of this invention the term "visible color change" is to be understood as a change of color that is - usually - detectable by the naked eye in the detection band when the mobilized (1-; or 2-)AAD-protein antibody conjugate in the sample contacts the (first or second) conjugate-binding protein in the (first or second) detection band. An example is the color change from white to a red color if the "conjugate" is comprising gold particles or from white to a blue color if the "conjugate" is comprising blue Latex particles.

In the context of this invention the term "D-dimer" is to be understood as a specific degradation product of cross-linked fibrin that is normally produced by plasmin hydrolysis. A cardiac "D-dimer" assay is used for detection of pulmonary embolism and deep vein thrombosis.

In the context of this invention the term "smooth muscle actin" is to be understood as an actin protein that is a globular multi-functional protein that form microfilaments and is involved in the contractile apparatus of smooth muscle.

In the context of this invention the term "smooth muscle myosin heavy chain" is to be understood as the heavy chain of myosin from smooth muscle cells,

In the context of this invention the term "cardiac myosin light chain" is to be understood as the light chain of myosin from heart muscle cells.

In the context of this invention the term "isozyme CK-MB" is to be understood as the creatinekinase creatine kinase myocardial band being the bound combination of two variants (isoenzymes CKM and CKB) of the enzyme phosphocreatine kinase. More precise, in the context of this invention the term "isozyme CK-MB" is to be understood as the creatine kinase creatine kinase myocardial band being the bound combination of two variants (isoenzymes CKM and CKB) of the enzyme phosphocreatine kinase.

In the context of this invention the term "creatinine phosphokinase (CK) or its MB isozyme" (also CPK) is to be understood as synonymous to creatine kinase (CK) wherein MB signifies "myocardial band". More precise, in the context of this invention the term "creatinine phosphokinase (CK) or its MB isozyme" (also CPK) is to be understood as synonymous to creatine kinase (CK) wherein MB signifies "myocardial band"

In the context of this invention the term "troponin T (TnT)" is to be understood as a part of troponin (or the troponin complex). It is integral to muscle contraction in skeletal muscle and cardiac muscle, but not smooth muscle. More precise, in the context of this invention the term "troponin T (TnT)" is to be understood as a part of troponin (or the troponin complex). It is integral to muscle contraction in skeletal muscle and cardiac muscle, but not smooth muscle.

In the context of this invention the term "troponin I (Tnl)" is to be understood as a part of troponin (or the troponin complex). It is integral to muscle contraction in skeletal muscle and cardiac muscle, but not smooth muscle. More precise, in the context of this invention the term "troponin I (Tnl)" is to be understood as a part of troponin (or the troponin complex). It is integral to muscle contraction in skeletal muscle and cardiac muscle, but not smooth muscle.

In the context of this invention the term "HMGI-C" (also HMGA2) is to be understood as a protein that belongs to the non-histone chromosomal high-mobility group (HMG) protein family. HMG proteins function as architectural factors and are essential components of the enhanceosome. It may act as a transcriptional regulating factor and its expression in adult tissues is commonly associated with both malignant and benign tumor formation. More precise, in the context of this invention the term "HMGI-C" (also HMGA2) is to be understood as a protein that belongs to the non-histone chromosomal high-mobility group (HMG) protein family. HMG proteins function as architectural factors and are essential components of the enhanceosome. It may act as a transcriptional regulating factor and its expression in adult tissues is commonly associated with both malignant and benign tumor formation.

In the context of this invention the term "Aggrecan" is to be understood as a proteoglycan able to form large aggregates in the cartilage tissue. It is am member of the chondroitin sulfate proteoglycan family and is an integral part of the extracellular matrix in cartilagenous tissue. "HMGI-C" (also HMGA2) is to be known to be present in isoforms.

In the context of this invention the term "soluble aortic elastin" is to be understood as and elastin that soluble and present at the aorta. Elastin is a key component of the extracellular matrix. It is highly elastic and present in connective tissue allowing many tissues in the body to resume their shape after stretching or contracting. Elastin is used in places where mechanical energy is required to be stored.

In the context of this invention the term "elastin degradation product" is to be understood as the degradation product of elastin.

In the context of this invention the term "human heart fatty acids binding protein" (hFABP) also known as mammary-derived growth inhibitor is a small cytoplasmic protein released from cardiac myocytes following an ischemic episode. It is involved in active fatty acid metabolism where it transports fatty acids from the cell membrane to mitochondria for oxidation.

In the context of this invention the term "acute myocardial infarction" is to be understood as a medical complication that occurs when blood flow decreases or stops to the coronary artery of the heart, causing damage to the heart muscle. It is also commonly known as a heart attack.

In the context of this invention the term "MI-protein" is to be understood as a protein indicative for acute myocardial infarction. Examples include creatinine phosphokinase (CK) or its MB isozyme, cardiac myosin light chain, troponin T (TnT) or troponin I (Tnl) and human heart fatty acids binding protein (MI-protein).

In the context of this invention the term "MI-detection band" is to be understood as a stretch on the strip into which the fluid sample or analyte together with the mobilized MI-protein antibody (sample) conjugate is transported via capillary flow. The MI-detection band is located substantially perpendicular to the direction of flow of the sample along the strip and is usually distanced from the (first r second) detection band. The MI-detection band does comprise (contain) a further conjugate-binding protein (see below). The further conjugate-binding protein is immobilized within the strip (the MI-detection band), preferably along a band (the Ml-detection band) located substantially perpendicular to the direction of flow of the sample along the strip. Thus, when the flow of the sample together with the mobilized MI-protein antibody being conjugated in the sample reaches the MI-detection band, it contacts (or - at least some MI-protein antibody conjugate - binds to) the further conjugate-binding protein comprised (contained) within the MI-detection band. This leads to that the presence of said MI-protein in the sample is indicated by a visible color change. This detection band can e.g. consist of polymers or paper, like sintered polymer, porous paper or a microstructured polymer or nitrocellulose.

In the context of this invention the term "wicking pad" is to be understood as a stretch on (or a part of) the strip into which the fluid sample or analyte is transported via capillary flow, e.g. in the strip. It is at the end of the strip (membrane) opposite the sample pad. It holds the fluids (sample or analyte) and reagents that have migrated via capillary action through the membrane (strip) and acts more or less as a waste container.

This wicking pad can e.g. consist of polymers or paper, like sintered polymer, porous paper or a microstructured polymer or nitrocellulose.

In the context of this invention the term "Protein G, Protein A or a Protein A/G fusion protein" is to be understood as a group of proteins that bind to (especially human) IgG, polyclonal or monoclonal antibodies as well as to IgA, IgE, IgM and IgD. Protein A/G is a Fusion protein that combines IgG binding domains of both Protein A and Protein G.

### Lateral Flow Device and Kits

With reference to the figures, Figure 1 shows an example of a Lateral Flow Device (1) according to the invention. The device (1) has a sample pad (2), a conjugate pad (3) including a mobilizable conjugate comprising an antibody protein binding to said AAD-Protein that has been conjugated to a detection agent, a membrane (4), and an optional wicking pad (7) for receiving and holding fluid that has travelled by capillary flow from the sample pad (2) and through the conjugate pad (3) and the membrane (4). A detection band (5) is shown which comprises an immobilized binding protein. Band (6) is an optional positive control band. The device (1) can further include a backing (8).

In general and as one aspect, the present invention provides a lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
wherein the 1-AAD-protein is HMGI-C.

In a preferred embodiment B of the lateral flow device according to the invention the 1-AAD-protein is HMGI-C and the lateral flow device further comprises a second protein antibody binding to a second protein potentially also released during said event leading to an acute aortic dissection (2-AAD-protein). Preferably, the 2-AAD-protein is selected from a group of biomarkers for AAD including D-dimer, and especially from aggrecan, smooth muscle myosin heavy chain, soluble aortic elastin or D-dimer. More prefeably, the 2-AAD-protein is D-Dimer.

Most preferably in this embodiment B, the 1-AAD-protein is HMGI-C and the 2-AAD-protein is D-Dimer.

This is a very preferred embodiment of the lateral flow device of the invention which thus comprises (at least) a 1^{st} anitibody to HMGI-C (the 1-AAD-protein) and a 2^{nd} antibody binding to D-Dimer (the X-AAD-protein). This combination allows a very good differentiation of AAD and MI with the combination of HMGI-C and D-Dimer strongly increasing the accuracy of the detection of AAD.

It is preferred if in the embodiment B of the preceding paragraph the lateral flow device according to the invention said second protein antibody to the 2-AAD-protein is conjugated to a second detection agent different from the one conjugated to said antibody to the 1-AAD-protein and said conjugate with the second protein antibody to the 2-AAD-protein is either located in said first conjugate pad or in a further conjugate pad; or
said second protein antibody to the 2-AAD-protein is conjugated to the same detection agent as the one conjugated to said antibody to the 1-AAD-protein and said conjugate with the second protein antibody to the 2-AAD-protein is either located in said first conjugate pad or in a further conjugate pad, preferably wherein the first conjugate-binding protein in the first detection band will, when the second mobilized 2-AAD-protein antibody conjugate in the sample contacts the first conjugate-binding protein in the first detection band, the presence of the 2-AAD-protein in the sample is indicated by a visible color change.

It is preferred if in these embodiments B of the preceding paragraph the lateral flow device according to the invention comprises a second detection band comprising a second conjugate-binding protein different from the first immobilized within the strip along a band located distanced from the first detection band substantially perpendicular to the direction of flow of the sample along the strip so that when the mobilized 2-AAD-protein antibody conjugate in the sample contacts the second conjugate-binding protein in the second detection band, the presence of the 2-AAD-protein in the sample is indicated by a visible color change.

In a preferred embodiment C of the lateral flow device according to the invention the lateral flow device further comprises one or more further protein antibody/ies binding to one or more further protein/s potentially also released during said event leading to an acute aortic dissection (X-AAD-protein/s), preferably wherein the 1-AAD-protein and the X-AAD-protein/s is/are selected from a group of biomarkers for AAD including aggrecan, smooth muscle myosin heavy chain, soluble aortic elastin or D-dimer.

In a preferred embodiment D of the lateral flow device according to the invention the 1-AAD-protein is HMGI-C and the lateral flow device further comprises one or more further protein antibody/ies binding to one or more further protein/s potentially also released during said event leading to an acute aortic dissection (X-AAD-protein/s), preferably wherein the X-AAD-protein/s is/are selected from a group of biomarkers for AAD including aggrecan, smooth muscle myosin heavy chain, soluble aortic elastin or D-dimer.

In a preferred embodiment C or D of the lateral flow device according to the invention said one or more further protein antibody/ies to the X-AAD-protein/s is/are conjugated to the same detection agent as the one conjugated to said antibody to the 1-AAD-protein and said conjugate with the one or more further protein antibody/ies to the X-AAD-protein/s is/are located in said first conjugate pad and the first conjugate-binding protein in the first detection band will when the one or more further mobilized X-AAD-protein/s antibody/ies conjugate/s in the sample contact/s the first conjugate-binding protein in the first detection band, the presence of the one or more X-AAD-protein/s in the sample is/are indicated by a visible color change.

As a general principal of the lateral flow device/s of the invention, proteins should be identified, whose blood (including Serum/Plasma) elevation/concentration differs between patients with aortic dissection and patients with myocardial infarction. Of note, MI and AAD often show the same symptoms, gold standard until today for MI diagnosis is based on "STE" on the 12-lead Electrocardiographic diagnostic.

In another (thus preferred) embodiment of the lateral flow device according to the invention, the lateral flow device further comprises at least one MI-protein antibody binding to a protein indicative for acute myocardial infarction (MI-protein) together with a separate Ml-detection band comprising a further conjugate-binding protein different from the first and second immobilized conjugate-binding protein immobilized within the strip along a band located substantially perpendicular to the direction of flow of the sample along the strip so that when an at least one mobilized MI-protein antibody sample conjugate in the sample contacts the further conjugate-binding protein in the MI-detection band the presence of said MI-protein in the sample is indicated by a visible color change, preferably wherein the MI-protein is selected from the group consisting of creatinine phosphokinase (CK) or its MB isozyme, cardiac myosin light chain, troponin T (TnT) (or also troponin I (Tnl)) and human heart fatty acids binding protein.

Very preferably in this embodiment, the MI-protein (to which the MI-protein antibody binds) is troponin, like T (TnT) or troponin I (Thl), or is troponin T (TnT). This allows a good differentiation of AAD and MI as MI and AAD often show the same symptoms,

Also very preferably in this embodiment, the MI-protein (to which the MI-protein antibodies bind) are troponin, like T (TnT) or troponin I (Thl), or is troponin T (TnT) and the other Ml-protein is creatinine phosphokinase (CK) and especially its MB isozyme (CK-MB). This combination allows a very good identification MI with the combination of troponin and CK-MB increasing the accuracy of the detection of myocardial infarction (e.g. to ≥ 91%). Thus, it also allows a good differentiation of AAD and MI as MI and AAD often show the same symptoms.

Most preferably in this embodiment, the lateral flow device according to this embodiment is defined by that:
- the lateral flow device further comprises a MI-protein antibody binding to the MI-protein being troponin, like T (TnT) or troponin I (Thl), or being troponin T (TnT);
- the 1-AAD-protein is HMGI-C; and
- the lateral flow device further comprises one further protein antibody binding to one further protein potentially also released during said event leading to an acute aortic dissection (X-AAD-protein) wherein the X-AAD-protein is D-dimer.

Thus, the lateral flow device comprises a 1^{st} anitibody to HMGI-C (the 1-AAD-protein), a 2^{nd} antibody binding to troponin (the MI-protein) and a 3^{rd} antibody binding to D-Dimer (the X-AAD-protein). This combination allows a very good differentiation of AAD and MI with the combination of HMGI-C and D-Dimer strongly increasing the accuracy of the detection of AAD.

Also most preferably in this embodiment, the lateral flow device according to this embodiment is defined by that:
- the lateral flow device further comprises two MI-protein antibodies with one antibody binding to the MI-protein troponin, like T (TnT) or troponin I (Thl) (or especially troponin T (TnT)), and the other antibody binding to the MI-protein creatinine phosphokinase (CK) and especially its MB isozyme (CK-MB);
- the 1-AAD-protein is HMGI-C; and
- the lateral flow device further comprises one further protein antibody binding to one further protein potentially also released during said event leading to an acute aortic dissection (X-AAD-protein) wherein the X-AAD-protein is D-dimer.

Thus, the lateral flow device of the invention comprises a 1^{st} anitibody to HMGI-C (the 1-AAD-protein), a 2^{nd} antibody binding to troponin (the MI-protein), a 3^{rd} antibody binding to D-Dimer (the X-AAD-protein) and a 4^{th} antibody binding to CK and especially its MB isozyme (CK-MB). This combination allows a very good differentiation of AAD and MI with the combination of HMGI-C and D-Dimer strongly increasing the accuracy of the detection of AAD and with the combination of troponin and CK-MB increasing the accuracy of the detection of myocardial infarction (e.g. to ≥ 91%).

In one aspect, the present invention provides a lateral flow device (A1) for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device further comprises a MI-protein antibody binding to a protein indicative for acute myocardial infarction (MI-protein) wherein the Ml-protein is troponin I.

This combination allows a very good differentiation of AAD and MI with the combination of HMGI-C an accurate detection of AAD and combined with troponin I it allows the detection of myocardial infarction.

It is preferred if said lateral flow device (A1) comprising the at least one MI-protein antibody binding to a troponin I (Tnl) further comprises a separate MI-detection band comprising a further conjugate-binding protein different from the first (and potentially second) immobilized conjugate-binding protein immobilized within the strip along a band located substantially perpendicular to the direction of flow of the sample along the strip so that when the mobilized MI-protein antibody sample conjugate in the sample contacts the further conjugate-binding protein in the MI-detection band the presence of said MI-protein in the sample is indicated by a visible color change.

It is also preferred if said lateral flow device (A1) further comprises one or more further protein antibody/ies binding to one or more further protein/s potentially also released during said event leading to an acute aortic dissection (X-AAD-protein/s), wherein the X-AAD-protein/s is/are selected from a group of biomarkers for AAD including aggrecan, smooth muscle myosin heavy chain, soluble aortic elastin or D-dimer, preferably is D-Dimer
or
wherein the X-AAD-proteins are D-Dimer and isozyme CK-MB;
   or
wherein the X-AAD-protein is D-Dimer.

In one other aspect, the present invention provides a lateral flow device (A2) for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device is arranged so that if the sample reaches the first conjugate pad, the fluid in said first conjugate pad shows or turns to an alkaline pH.

As can be seen in Example 5 and exemplified there with 3 protein antibodies binding to HMGI-C, it is surprisingly important that an alkaline pH is present in the conjugate pad to achieve a successful incubation for determining HMGI-C in a bodily fluid, like blood.

It is preferred if regarding said lateral flow device (A2) the alkaline pH is ≥ 7.5, preferably is between 8.0 and 10.0, more preferably is between 8.0 and 9.5 or between 8.5 and 9.5.

Accordingly, it would be a very preferred embodiment of said lateral flow device (A2) if the lateral flow device is arranged so that if the sample reaches the first conjugate pad, the fluid in said first conjugate pad shows or turns to an alkaline pH of between 8.0 and 10.0 or between 8.5 and 9.5

It is preferred if regarding said lateral flow device (A2) the detection agent conjugated to the antibody is colloidal metallic gold.

Accordingly, it would be another very preferred embodiment of said lateral flow device (A2) if the lateral flow device is arranged so that if the sample reaches the first conjugate pad, the fluid in said first conjugate pad shows or turns to an alkaline pH of between 8.0 and 10.0 or between 8.5 and 9.5, and when the detection agent conjugated to the antibody is colloidal metallic gold.

These 2 embodiments are all even closer to the conditions of Example 5.

In one other aspect, the present invention provides a lateral flow device (A3) for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device comprises an alkaline chemical buffer.

As can be seen in Example 5 and exemplified there with 3 protein antibodies binding to HMGI-C, it is surprisingly important to achieve an alkaline pH by a buffer in the lateral flow device to achieve a successful detection of HMGI-C in a bodily fluid, like blood.

It is preferred if regarding said lateral flow device (A3) the lateral flow device comprises said alkaline chemical buffer in at least one of the first conjugate pad, the strip between the sample pad and the first conjugate pad, or in the sample pad, preferably in the first conjugate pad. Accordingly, it would be another very preferred embodiment of said lateral flow device (A3) if the lateral flow device is arranged so that it comprises said alkaline chemical buffer in the first conjugate pad

It is preferred if regarding said lateral flow device (A3) if the buffer if contacted with the sample of bodily fluid is set to show a pH ≥ 7.5, preferably between 8.0 and 10.0, more preferably between 8.0 and 9.5, between 8.5 and 9.5.

Accordingly, it would be another very preferred embodiment of said lateral flow device (A3) if the lateral flow device is arranged so that it comprises said alkaline chemical buffer in the first conjugate pad and if the buffer if contacted with the sample of bodily fluid is set to show a pH between 8.0 and 10.0 or between 8.5 and 9.5.

It is preferred if regarding said lateral flow device (A3) if the detection agent conjugated to the antibody is colloidal metallic gold.

Accordingly, it would be another very preferred embodiment of said lateral flow device (A3) if the lateral flow device is arranged so that it comprises said alkaline chemical buffer in the first conjugate pad, if the buffer if contacted with the sample of bodily fluid is set to show a pH between 8.0 and 10.0 or between 8.5 and 9.5 and if the detection agent conjugated to the antibody is colloidal metallic gold.

These 3 embodiments are all even closer to the conditions of Example 5.

In one other aspect, the present invention provides a method (B1) for the immunoassay determination of acute aortic dissection (AAD) in a patient, said method comprising:
a) incubating a bodily fluid sample of the patient with
   ∘ at least one antibody to a first protein released during an event leading to an acute aortic dissection (1-AAD-protein) and
   ∘ a binding partner B for either 1-AAD-protein or the antibody
   wherein either the antibody or the binding partner B is conjugated to a detection agent,
   to form an immunological complex containing the detection agent;
b) isolating the immunological complex from the remaining sample; and
c) determining the detection agent either in the isolated complex or in the remaining sample as an indicator of the 1-AAD-protein in the sample;

thereby determining the occurrence of AAD in the patient,
wherein the 1-AAD-protein is HMGI-C; and
wherein step a) of incubating is done at an alkaline pH.

As can be seen in Example 5 and exemplified there with 3 protein antibodies binding to HMGI-C, it is surprisingly important to install an alkaline pH to achieve a successful incubation for determining AAD by detection of HMGI-C in a bodily fluid, like blood.

In general, it cannot be overestimated that it is a real breakthrough using HMGI-C as the biomarker for determining or identifying AAD and this does fulfill a long-standing need. Even though Belge et al. mention HMGA2/HMGI-C in the context of AAD, the article is focused on tissue and the expression levels seen. The importance of the finding that HMGA2/HMGI-C is released into the blood by the AAD event and the finding that this marker can be detected there (in the blood) as such an indicator is not disclosed by Belge. This is extremely important for the cardiologist and for emergency treatment, given the lethality of AAD and the need for immediate action in this - often overlooked - condition. As said above, there is currently no easy way for identifying AAD in a cardiac emergency situation. Just the fact that CN 105 259 353 A mentions ST2 as a (potential) biomarker in the blood over 8 years ago does not indicate such a breakthrough and the applicant/inventor is not aware of finding this marker in any wider use. And - as said above - Belge does not mention HMGA2/HMGI-C as a biomarker in the blood.

It is preferred if regarding said method (B1) the alkaline pH is pH ≥ 7.5, preferably between 8.0 and 10.0, more preferably between 8.0 and 9.5, between 8.5 and 9.5.

Accordingly, it would be a very preferred embodiment of said method (B1) if the alkaline pH is between 8.0 and 10.0 or between 8.5 and 9.5.

It is preferred if regarding said method (B1) the detection agent is colloidal metallic gold.

Accordingly, it would be a very preferred embodiment of said method (B1) if the alkaline pH is between 8.0 and 10.0 or between 8.5 and 9.5, and when the detection agent is colloidal metallic gold.

These two embodiments are even closer to the conditions of Example 5.

In one other aspect, the present invention provides a method (B2) for the immunoassay determination of acute aortic dissection (AAD) in a patient, said method comprising:
a) incubating a bodily fluid sample of the patient with
   ∘ at least one antibody to a first protein released during an event leading to an acute aortic dissection (1-AAD-protein) and
   ∘ a binding partner B for either 1-AAD-protein or the antibody
   wherein either the antibody or the binding partner B is conjugated to a detection agent,
   to form an immunological complex containing the detection agent;
b) isolating the immunological complex from the remaining sample; and
c) determining the detection agent either in the isolated complex or in the remaining sample as an indicator of the 1-AAD-protein in the sample;
d) comparing the said value of said 1-AAD-protein with a known reference of the respective bodily fluid of a human without acute aortic dissection;

thereby determining the occurrence of AAD in the patient,
wherein the 1-AAD-protein is HMGI-C.

As already said above and repeated here, it cannot be overestimated that it is a real breakthrough using HMGI-C as the biomarker for determining or identifying AAD and this does fulfill a long-standing need. Even though Belge et al. mention HMGA2/HMGI-C in the context of AAD, the article is focused on tissue and the expression levels seen. The importance of the finding that HMGA2/HMGI-C is released into the blood by the AAD event and the finding that this marker can be detected there (in the blood) as such an indicator is not disclosed by Belge. This is extremely important for the cardiologist and for emergency treatment, given the lethality of AAD and the need for immediate action in this - often overlooked - condition. As said above, there is currently no easy way for identifying AAD in a cardiac emergency situation. Just the fact that CN 105 259 353 A mentions ST2 as a (potential) biomarker in the blood over 8 years ago does not indicate such a breakthrough and the applicant/inventor is not aware of finding this marker in any wider use. And - as said above - Belge does not mention HMGA2/HMGI-C as a biomarker in the blood.

It is preferred if regarding said method (B2) the bodily fluid is blood, especially is full blood, blood plasma and/or blood serum.

In one other aspect, the present invention provides a method (B3) for the detection of acute aortic dissection (AAD) by detecting HMGI-C in a sample of bodily fluid of a subject, the method comprising:
1) contacting the sample with a protein antibody binding to HMGI-C and thus able to form HMGI-C/Antibody-conjugates;
2) detecting the presence and quantifying the amount of said HMGI-C/Antibody-conjugate in the sample following step 1); and
3) comparing the value obtained in step 3) with a known reference.

As said above, it cannot be overestimated that it is a real using HMGI-C as the biomarker for determining or identifying AAD is a breakthrough in cardiology.

It is preferred if regarding said method (B3) the bodily fluid is blood, especially is full blood, blood plasma and/or blood serum.

The invention is further described by way of Embodiments. It will be appreciated that the invention as claimed is not intended to be limited in any way by these Embodiments.

### EMBODIMENTS:

### EMBODIMENT A [Lateral Flow assay with HMGI-C + Troponin I]:

**A01)** A lateral flow device (suitable) for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein with that protein antibody having been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C and (thus) the lateral flow device comprises said protein antibody binding to HMGI-C (said 1-AAD-protein); and
wherein the lateral flow device further comprises a MI-protein antibody binding to a protein indicative for acute myocardial infarction (MI-protein) wherein the Ml-protein is troponin I (Tnl).

This combination allows a very good differentiation of AAD and MI with the combination of HMGI-C an accurate detection of AAD and combined with troponin I it allows the detection of myocardial infarction.

**A02)** The lateral flow device according to EMBODIMENT **A01),** wherein the lateral flow device further comprises one or more further protein antibody/ies binding to one or more further protein/s potentially also released during said event leading to an acute aortic dissection (X-AAD-protein/s), wherein the X-AAD-protein/s is/are selected from a group of biomarkers for AAD including aggrecan, smooth muscle myosin heavy chain, soluble aortic elastin or D-dimer, preferably is D-Dimer
or
wherein the X-AAD-proteins are D-Dimer and isozyme CK-MB;
   or
wherein the X-AAD-protein is D-Dimer.

**A03)** The lateral flow device according to EMBODIMENTS **A01)** or **A02),** wherein the lateral flow device shows an alkaline pH in the first conjugate pad, which comprises the protein antibody binding to HMGI-C (said 1-AAD-protein).

**A04)** The lateral flow device according to EMBODIMENT **A03),** wherein the alkaline pH is ≥ 7.5, preferably is between 8.0 and 10.0, more preferably is between 8.0 and 9.5 or between 8.5 and 9.5.

**A05)** The lateral flow device according to any one of EMBODIMENTS **A01)** to **A04),** wherein the lateral flow device comprises the at least one MI-protein antibody binding to a troponin I (Tnl) further comprises a separate MI-detection band comprising a further conjugate-binding protein different from the first (and potentially second) immobilized conjugate-binding protein immobilized within the strip along a band located substantially perpendicular to the direction of flow of the sample along the strip so that when the mobilized MI-protein antibody sample conjugate in the sample contacts the further conjugate-binding protein in the MI-detection band the presence of said MI-protein in the sample is indicated by a visible color change.

**A06)** The lateral flow device according to any one of EMBODIMENTS **A01)** to **A05),** wherein the protein antibody binding to said 1-AAD-protein, HMGI-C, is
- an antibody (I) comprising a heavy chain variable region with at least 95%, 99% or 100% sequence identity to SEQ ID NO: 1 and a light chain variable region with at least 95%, 99% or 100% sequence identity to SEQ ID NO: 6;
   OR
- an antibody (II) comprising a heavy chain variable region with the amino acid sequence of SEQ ID NO: 11 or with at least 95% or 99% sequence identity to SEQ ID NO: 11 and a light chain variable region with the amino acid sequence of SEQ ID NO: 16 or with at least 95% or 99% sequence identity to SEQ ID NO: 16;
   OR
- an antibody (III) comprising a heavy chain variable region with the amino acid sequence of SEQ ID NO: 11 or with at least 95% or 99% sequence identity to SEQ ID NO: 21 and a light chain variable region with the amino acid sequence of SEQ ID NO: 16 or with at least 95% or 99% sequence identity to SEQ ID NO: 26.

**A07)** The lateral flow device according to any one of EMBODIMENTS **A01)** or **A05),** wherein the protein antibody binding to said 1-AAD-protein, HMGI-C, comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

**A08)** The lateral flow device according to any one of EMBODIMENTS **A01)** to **A07),** wherein the lateral flow device is arranged so that if the sample reaches the first conjugate pad, the fluid in said first conjugate pad shows or turns to an alkaline pH.

**A09)** The lateral flow device according to EMBODIMENT **A08),** wherein the alkaline pH is ≥ 7.5, preferably is between 8.0 and 10.0, more preferably is between 8.0 and 9.5 or between 8.5 and 9.5.

Accordingly, it would be a very preferred for EMBODIMENT **A)** if the lateral flow device is arranged so that if the sample reaches the first conjugate pad, the fluid in said first conjugate pad shows or turns to an alkaline pH of between 8.0 and 10.0 or between 8.5 and 9.5

**A10)** The lateral flow device according to EMBODIMENTS **A07)** and **A09),** wherein the alkaline pH is provided by choice of the material of the device and/or a chemical buffer comprised in the lateral flow device.

**A11)** The lateral flow device according to any one of EMBODIMENTS **A01)** to **A10),** wherein the device comprises an alkaline buffer material, preferably in the part of the device comprising the protein antibody binding to the antigen HMGI-C.

**A12)** The lateral flow device according to EMBODIMENT **A11),** wherein the buffer is selected to show a pH ≥ 7.5, preferably a pH between 8.0 and 10.0, more preferably a pH between 8.0 and 9.5, or a pH between 8.5 and 9.5, if contacted with a fluid, preferably with bodily fluid, most preferably with blood, like full blood, blood plasma or blood serum.

**A13)** The lateral flow device according to any one of EMBODIMENTS **A01)** to **A12),** wherein the protein antibody binding to the antigen troponin I has been conjugated to a detection agent.

**A14)** The lateral flow device according to EMBODIMENT **A13),** wherein the detection agent conjugated to the antibody binding to the antigen HMGI-C is different from the detection agent conjugated to the antibody binding to the antigen troponin.

**A15)** The lateral flow device according to EMBODIMENT **A13)**, wherein the detection agent conjugated to the antibody binding to the antigen HMGI-C is the same detection agent as the one conjugated to the antibody binding to the antigen troponin.

**A16)** The lateral flow device according to any one of EMBODIMENTS **A01)** to **A15)**, wherein the detection agent conjugated to the antibody binding to the antigen HMGI-C is colloidal (metallic) gold.

In line with that, it would be very preferred for EMBODIMENT **A)** if the lateral flow device is arranged so that if the sample reaches the first conjugate pad, the fluid in said first conjugate pad shows or turns to an alkaline pH of between 8.0 and 10.0 or between 8.5 and 9.5, and when the detection agent conjugated to the antibody is colloidal metallic gold.

**A17)** The lateral flow device according to any one of EMBODIMENTS **A01)** to **A15)**, wherein the respective detection agent conjugated to said antibodies is independently selected from the group comprising metallic nanoparticles or nanoshells, non-metallic nanoparticles or nanoshells, enzymes, colloidal gold and fluorescent molecules, preferably wherein the detection agent conjugated to the antibodies comprises nanoparticles or nanoshells of metallic gold or colloidal gold.

**A18)** The lateral flow device according to any one of EMBODIMENTS **A01)** to **A17)**, wherein the acute aortic dissection (AAD) is acute aortic syndrome which comprises aortic dissection, including acute type A aortic dissection, and/or one or more of the following aortic dissections/acute aortic syndroms:
- Penetrating Aortic Ulcer and Intramural Hematoma
- Thoracic Aortic Aneurysm
- Chronic Dissection-Indications with Treatment with Branched and Fenestrated Stent-grafts
- Different Therapeutic Modalities for Aortic Arch Dissection Combined with Kommerell's Diverticulum
- Secondary Interventions After Endovascular Repair of Aortic Dissections
- False Aneurysms Complicating Internal Carotid Artery Dissection
- Established Indications for the Invasive Treatment of a Thoracic Aortic Aneurysm
- Aortic Arch Dissection: A Controversy of Classification
- Treatment Algorithms for Patients with (Sub)acute Type B Aortic Dissections
- Descending Thoracic Aortic Dissections
- Differences in Aortopathy in Patients with a Bicuspid Aortic Valve with or Without Aortic Coarctation
- Angina Chest pain
- Dyspnea Accompanied by Hypertension Crisis
- Sudden Coma or
- Shock of Unknown Etiology.

**A19)** The lateral flow device according to any one of EMBODIMENTS **A01)** to **A18)**, wherein the lateral flow device according to any one of the proceeding claims, wherein the conjugate-binding protein is an FC-binding protein, preferably wherein the FC-binding protein is Protein G, Protein A or a Protein A/G fusion protein.

**A20)** The lateral flow device according to any one of EMBODIMENTS **A01)** to **A19)**, wherein the lateral flow device according to any one of the preceding claims, further comprising a control line located substantially perpendicular to the direction of flow of the sample along the strip, preferably wherein the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line, preferably wherein deposited at the control line is an antibody capable of binding to the mobilized immunoglobin conjugate as it crosses the control line, with optionally said binding at the control line being indicated by a visible color change; even more preferably wherein the immunoglobulin in the conjugate is from an animal species other than the mammal species from which the sample of bodily fluid is derived.

### EMBODIMENT B [Lateral Flow assay with alkaline conjugate pad]:

**B01)** A lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device is arranged so that if the sample reaches the first conjugate pad, the fluid in said first conjugate pad shows or turns to an alkaline pH.

**B02)** The lateral flow device according to EMBODIMENT **B01),** wherein the alkaline pH is ≥ 7.5, preferably is between 8.0 and 10.0, more preferably is between 8.0 and 9.5 or between 8.5 and 9.5.

**B03)** The lateral flow device according to EMBODIMENTS **B01)** or **B02),** wherein the detection agent conjugated to the antibody is colloidal metallic gold.

**B04)** The lateral flow device according to any one of EMBODIMENTS **B01)** to **B03),** wherein the protein antibody binding to said 1-AAD-protein, HMGI-C, is
- an antibody (I) comprising a heavy chain variable region with at least 95%, 99% or 100% sequence identity to SEQ ID NO: 1 and a light chain variable region with at least 95%, 99% or 100% sequence identity to SEQ ID NO: 6;
   OR
- an antibody (II) comprising a heavy chain variable region with the amino acid sequence of SEQ ID NO: 11 or with at least 95% or 99% sequence identity to SEQ ID NO: 11 and a light chain variable region with the amino acid sequence of SEQ ID NO: 16 or with at least 95% or 99% sequence identity to SEQ ID NO: 16;
   OR
- an antibody (III) comprising a heavy chain variable region with the amino acid sequence of SEQ ID NO: 11 or with at least 95% or 99% sequence identity to SEQ ID NO: 21 and a light chain variable region with the amino acid sequence of SEQ ID NO: 16 or with at least 95% or 99% sequence identity to SEQ ID NO: 26.

**B05)** The lateral flow device according to any one of EMBODIMENTS **B01)** or **B03)**, wherein the protein antibody binding to said 1-AAD-protein, HMGI-C, comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

**B06)** The lateral flow device according to any one of EMBODIMENTS **B01)** to **B05)**, wherein the acute aortic dissection (AAD) is acute aortic syndrome which comprises aortic dissection, including acute type A aortic dissection, and/or one or more of the following aortic dissections/acute aortic syndroms:
- Penetrating Aortic Ulcer and Intramural Hematoma
- Thoracic Aortic Aneurysm
- Chronic Dissection-Indications with Treatment with Branched and Fenestrated Stent-grafts
- Different Therapeutic Modalities for Aortic Arch Dissection Combined with Kommerell's Diverticulum
- Secondary Interventions After Endovascular Repair of Aortic Dissections
- False Aneurysms Complicating Internal Carotid Artery Dissection
- Established Indications for the Invasive Treatment of a Thoracic Aortic Aneurysm
- Aortic Arch Dissection: A Controversy of Classification
- Treatment Algorithms for Patients with (Sub)acute Type B Aortic Dissections
- Descending Thoracic Aortic Dissections
- Differences in Aortopathy in Patients with a Bicuspid Aortic Valve with or Without Aortic Coarctation
- Angina Chest pain
- Dyspnea Accompanied by Hypertension Crisis
- Sudden Coma or
- Shock of Unknown Etiology.

**B07)** The lateral flow device according to any one of EMBODIMENTS **B01)** to **B06)**, wherein the lateral flow device according to any one of the proceeding claims, wherein the conjugate-binding protein is an FC-binding protein, preferably wherein the FC-binding protein is Protein G, Protein A or a Protein A/G fusion protein.

**B08)** The lateral flow device according to any one of EMBODIMENTS **B01)** to **B07)**, wherein the lateral flow device according to any one of the preceding claims, further comprising a control line located substantially perpendicular to the direction of flow of the sample along the strip, preferably wherein the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line, preferably wherein deposited at the control line is an antibody capable of binding to the mobilized immunoglobin conjugate as it crosses the control line, with optionally said binding at the control line being indicated by a visible color change; even more preferably wherein the immunoglobulin in the conjugate is from an animal species other than the mammal species from which the sample of bodily fluid is derived.

**B09)** The lateral flow device according to any one of EMBODIMENTS **B01)** to **B08),** wherein the lateral flow device further comprises a MI-protein antibody binding to a protein indicative for acute myocardial infarction (MI-protein) wherein the MI-protein is troponin.

**B10)** The lateral flow device according to EMBODIMENT **B09),** wherein the troponin is troponin T (TnT) or troponin I (Tnl), especially is troponin I (Tnl).

**B11)** The lateral flow device according to any one of EMBODIMENTS **B01)** to **B10),** wherein the lateral flow device further comprises one further protein antibody binding to one further protein potentially also released during said event leading to an acute aortic dissection (2-AAD-protein), wherein the 2-AAD-protein is D-Dimer.

**B12)** The lateral flow device according to any one of EMBODIMENTS **B01)** to **B11),** wherein the lateral flow device further comprises one further protein antibody binding to one further protein potentially also released during said event leading to an acute aortic dissection (2-AAD-protein), wherein the 2-AAD-protein is creatinine phosphokinase (CK) or preferably the creatinine phosphokinase MB isozyme (CK-MB).

### EMBODIMENT C [Lateral Flow assay with alkaline buffer]:

**C01)** A lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C; and
wherein the lateral flow device comprises a chemical buffer, the fluid in said first conjugate pad shows or turns to an alkaline pH first conjugate pad.
wherein the lateral flow device comprises an alkaline chemical buffer.

**C02)** The lateral flow device according to EMBODIMENT **C01),** wherein the lateral flow device comprises said alkaline chemical buffer in at least one of the first conjugate pad, the strip between the sample pad and the first conjugate pad, or in the sample pad, preferably in the first conjugate pad.

**C03)** The lateral flow device according to EMBODIMENTS **C01)** or **C02),** wherein the buffer if contacted with the sample of bodily fluid is set to show a pH ≥ 7.5, preferably between 8.0 and 10.0, more preferably between 8.0 and 9.5, between 8.5 and 9.5.

**C04)** The lateral flow device according to any one of EMBODIMENTS **C01)** to **C03),** wherein the detection agent conjugated to the antibody is colloidal metallic gold.

**C05)** The lateral flow device according to any one of EMBODIMENTS **C01)** to **C04),** wherein the protein antibody binding to said 1-AAD-protein, HMGI-C, is
- an antibody (I) comprising a heavy chain variable region with at least 95%, 99% or 100% sequence identity to SEQ ID NO: 1 and a light chain variable region with at least 95%, 99% or 100% sequence identity to SEQ ID NO: 6;
   OR
- an antibody (II) comprising a heavy chain variable region with the amino acid sequence of SEQ ID NO: 11 or with at least 95% or 99% sequence identity to SEQ ID NO: 11 and a light chain variable region with the amino acid sequence of SEQ ID NO: 16 or with at least 95% or 99% sequence identity to SEQ ID NO: 16;
   OR
- an antibody (III) comprising a heavy chain variable region with the amino acid sequence of SEQ ID NO: 11 or with at least 95% or 99% sequence identity to SEQ ID NO: 21 and a light chain variable region with the amino acid sequence of SEQ ID NO: 16 or with at least 95% or 99% sequence identity to SEQ ID NO: 26.

**C06)** The lateral flow device according to any one of EMBODIMENTS **C01)** or **C04),** wherein the protein antibody binding to said 1-AAD-protein, HMGI-C, comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

**C07)** The lateral flow device according to any one of EMBODIMENTS **C01)** to **C06),** wherein the acute aortic dissection (AAD) is acute aortic syndrome which comprises aortic dissection, including acute type A aortic dissection, and/or one or more of the following aortic dissections/acute aortic syndroms:
- Penetrating Aortic Ulcer and Intramural Hematoma
- Thoracic Aortic Aneurysm
- Chronic Dissection-Indications with Treatment with Branched and Fenestrated Stent-grafts
- Different Therapeutic Modalities for Aortic Arch Dissection Combined with Kommerell's Diverticulum
- Secondary Interventions After Endovascular Repair of Aortic Dissections
- False Aneurysms Complicating Internal Carotid Artery Dissection
- Established Indications for the Invasive Treatment of a Thoracic Aortic Aneurysm
- Aortic Arch Dissection: A Controversy of Classification
- Treatment Algorithms for Patients with (Sub)acute Type B Aortic Dissections
- Descending Thoracic Aortic Dissections
- Differences in Aortopathy in Patients with a Bicuspid Aortic Valve with or Without Aortic Coarctation
- Angina Chest pain
- Dyspnea Accompanied by Hypertension Crisis
- Sudden Coma or
- Shock of Unknown Etiology.

**C08)** The lateral flow device according to any one of EMBODIMENTS **C01)** to **C07),** wherein the lateral flow device according to any one of the proceeding claims, wherein the conjugate-binding protein is an FC-binding protein, preferably wherein the FC-binding protein is Protein G, Protein A or a Protein A/G fusion protein.

**C09)** The lateral flow device according to any one of EMBODIMENTS **C01)** to **C08),** wherein the lateral flow device according to any one of the preceding claims, further comprising a control line located substantially perpendicular to the direction of flow of the sample along the strip, preferably wherein the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line, preferably wherein deposited at the control line is an antibody capable of binding to the mobilized immunoglobin conjugate as it crosses the control line, with optionally said binding at the control line being indicated by a visible color change; even more preferably wherein the immunoglobulin in the conjugate is from an animal species other than the mammal species from which the sample of bodily fluid is derived.

**C10)** The lateral flow device according to any one of EMBODIMENTS **C01)** to **C09),** wherein the lateral flow device further comprises a MI-protein antibody binding to a protein indicative for acute myocardial infarction (MI-protein) wherein the MI-protein is troponin.

**C11)** The lateral flow device according to EMBODIMENT **C10),** wherein the troponin is troponin T (TnT) or troponin I (Tnl), especially is troponin I (Tnl).

**C12)** The lateral flow device according to any one of EMBODIMENTS **C01)** to **C11),** wherein the lateral flow device further comprises one further protein antibody binding to one further protein potentially also released during said event leading to an acute aortic dissection (2-AAD-protein), wherein the 2-AAD-protein is D-Dimer.

**C13)** The lateral flow device according to any one of EMBODIMENTS **C01)** to **C12),** wherein the lateral flow device further comprises one further protein antibody binding to one further protein potentially also released during said event leading to an acute aortic dissection (2-AAD-protein), wherein the 2-AAD-protein is creatinine phosphokinase (CK) or preferably the creatinine phosphokinase MB isozyme (CK-MB).

### EMBODIMENT D [Method for the determination of acute aortic dissection using a Lateral Flow assay with HMGI-C]:

**D00)** A method for the determination of acute aortic dissection (AAD) in a patient, said method comprising
a) placing the sample onto a sample pad of a lateral flow device, the device being a lateral flow device of any of the Embodiments **A), B), C)** and**H).**

**D01)** A method for the determination of acute aortic dissection (AAD) in a patient, said method comprising
a) placing the sample onto a sample pad of a lateral flow device, the device comprising:
   i) a strip formed of a material enabling capillary flow of fluid along a portion of the strip;
   ii) a sample pad located proximal to one end of the strip for receiving the sample of the bodily fluid;
   iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
   iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change;
b) interrogating the detection region for the presence of label particles to detect whether said AAD protein is present in the sample.
wherein the 1-AAD-protein is HMGI-C.

As said various times above, it is a real breakthrough using HMGI-C as the biomarker for determining or identifying AAD and this does fulfill a long-standing need. Even though Belge et al. mention HMGA2/HMGI-C in the context of AAD, the article is focused on tissue and the expression levels seen. The importance of the finding that HMGA2/HMGI-C is released into the blood by the AAD event and the finding that this marker can be detected there (in the blood) as such an indicator is not disclosed by Belge. This is extremely important for the cardiologist and for emergency treatment, given the lethality of AAD and the need for immediate action in this - often overlooked - condition. As said above, there is currently no easy way for identifying AAD in a cardiac emergency situation. Just the fact that CN 105 259 353 A mentions ST2 as a (potential) biomarker in the blood over 8 years ago does not indicate such a breakthrough and the applicant/inventor is not aware of finding this marker in any wider use. And - as said above - Belge does not mention HMGA2/HMGI-C as a biomarker in the blood.

**D02)** The method according to EMBODIMENT **D01)** wherein the lateral flow device comprises an alkaline chemical buffer.

**D03)** The lateral flow device according to EMBODIMENT **D02),** wherein the lateral flow device comprises said alkaline chemical buffer in at least one of the first conjugate pad, the strip between the sample pad and the first conjugate pad, or in the sample pad, preferably in the first conjugate pad.

**D04)** The lateral flow device according to EMBODIMENTS **002)** or **D03),** wherein the buffer if contacted with the sample of bodily fluid is set to show a pH ≥ 7.5, preferably between 8.0 and 10.0, more preferably between 8.0 and 9.5, between 8.5 and 9.5.

**D05)** The method according to EMBODIMENT **D01)** wherein the lateral flow device in this method is a lateral flow device of any of the EMBODIMENTS **A, B, C** or **H** described herein.

**D06)** The method according to any one of EMBODIMENTS **D01)** to **D05),** wherein the step of interrogating the detection region for the presence of label particles to detect whether said AAD protein is present in the sample involves comparing the result with a standard reference which is indicative for the presence of acute aortic dissection (AAD) in said patient.

**007)** The method according to EMBODIMENT **D06)** wherein if the step of interrogating the detection region is indicating the presence of acute aortic dissection (AAD) in said patient, this is triggering surgical treatment of the ascending aorta in said patient.

**D08)** The method according to any one of EMBODIMENTS **D01)** to **D07),** wherein the sample is blood, especially full blood, blood plasma or blood serum.

### EMBODIMENT E [Immunoassay determination of acute aortic dissection (AAD) WITH ALKALINE PH]:

**E00)** A method for the immunoassay determination of acute aortic dissection (AAD) in a patient, said method comprising:
a) incubating a body fluid sample of the patient with
   a. at least one antibody to a first protein released during an event leading to an acute aortic dissection (1-AAD-protein) and
   b. a binding partner B for either 1-AAD-protein or the antibody

   wherein either the antibody or the binding partner B is conjugated to a detection agent,
   to form an immunological complex containing the detection agent;
b) isolating the immunological complex from the remaining sample; and
c) determining the detection agent either in the isolated complex or in the remaining sample as an indicator of the 1-AAD-protein in the sample;

thereby determining the occurrence of AAD in the patient,
wherein the 1-AAD-protein is HMGI-C.

In general, it cannot be overestimated that it is a real breakthrough using HMGI-C as the biomarker for determining or identifying AAD and this does fulfill a long-standing need. Even though Belge et al. mention HMGA2/HMGI-C in the context of AAD, the article is focused on tissue and the expression levels seen. The importance of the finding that HMGA2/HMGI-C is released into the blood by the AAD event and the finding that this marker can be detected there (in the blood) as such an indicator is not disclosed by Belge. This is extremely important for the cardiologist and for emergency treatment, given the lethality of AAD and the need for immediate action in this - often overlooked - condition. As said above, there is currently no easy way for identifying AAD in a cardiac emergency situation. Just the fact that CN 105 259 353 A mentions ST2 as a (potential) biomarker in the blood over 8 years ago does not indicate such a breakthrough and the applicant/inventor is not aware of finding this marker in any wider use. And - as said above - Belge does not mention HMGA2/HMGI-C as a biomarker in the blood.

In general, all these method for the immunoassay determination of acute aortic dissection (AAD) in a patient of EMBODIMENT E) can also be understood as a method/methods for providing a first orientation for treating a patient in a potential cardiac emergency including detection of the level of HMGI-C in a body fluid of a patient.

**E01)** A method for the immunoassay determination of acute aortic dissection (AAD) in a patient, said method comprising:
a) incubating a body fluid sample of the patient with
   - at least one antibody to a first protein released during an event leading to an acute aortic dissection (1-AAD-protein) and
   - a binding partner B for either 1-AAD-protein or the antibody

   wherein either the antibody or the binding partner B is conjugated to a detection agent,
   to form an immunological complex containing the detection agent;
b) isolating the immunological complex from the remaining sample; and
c) determining the detection agent either in the isolated complex or in the remaining sample as an indicator of the 1-AAD-protein in the sample;

thereby determining the occurrence of AAD in the patient,
wherein the 1-AAD-protein is HMGI-C; and
wherein step a) of incubating is done at an alkaline pH.

As can be seen in Example 5 and exemplified there with 3 protein antibodies binding to HMGI-C, it is surprisingly important to install an alkaline pH to achieve a successful incubation for determining AAD by detection of HMGI-C in a bodily fluid, like blood.

There are many options for exercising this method of immunoassay determination. One of these is the protein multiplex technique, especially drawn to the apparatus Luminex^{®} with which an analysis of blood using the herein-described antibodies could be done.

**E02**) The method according to EMBODIMENT **E01),** wherein the alkaline pH is pH ≥ 7.5, preferably between 8.0 and 10.0, more preferably between 8.0 and 9.5, between 8.5 and 9.5.

**E03S)** The method according to EMBODIMENTS **E01)** or **E02),** wherein the detection agent is colloidal metallic gold.

In the line of this, it is preferred if the method of EMBODIMENT **E** the alkaline pH is between 8.0 and 10.0, or between 8.5 and 9.5 and the detection agent is colloidal metallic gold.

**E04)** The method according to any one of EMBODIMENTS **E00)** or **E01)** to **E03S)** wherein if the method is indicative for the presence of acute aortic dissection (AAD) in said patient, this is triggering surgical treatment of the ascending aorta in said patient.

**E03S)** The method according to any one of EMBODIMENTS **E00)** or **E01)** to **E04),** wherein the sample is blood, especially full blood, blood plasma or blood serum.

**E06)** The method according to any one of EMBODIMENTS **E00)** to **E04),** wherein the method comprises the following additional steps:
d) incubating a body fluid sample of said patient with
   - at least one antibody to a protein indicative for acute myocardial infarction (MI-protein) and
   - a binding partner C for either said MI-protein or the antibody to said MI-protein

   wherein either the antibody to said MI-protein or the binding partner C is conjugated to a detection agent,
   to form an immunological MI-complex containing the detection agent;
e) isolating the immunological MI-complex from the remaining sample; and
f) determining the detection agent either in the isolated MI-complex or in the remaining sample as an indicator of the MI-protein in the sample
   thereby determining the potential occurrence of myocardial infarction in the patient, wherein the MI-protein is troponin, troponin T(TnT) or troponin I (Tnl), preferably troponin I (Tnl);
   wherein
      - if the results of the method indicate the presence of the 1-AAD-protein in the sample and the results of the method indicate no presence of the MI-protein in the sample, it strengthens the assumption of the presence of AAD in the patient;
      - if the results of the method indicate the presence of the MI-protein in the sample and the results of the method indicate no presence of the 1-AAD-protein in the sample, it is an indication of no presence of AAD in the patient but of myocardial infarction.

**E07)** The method according to any one of EMBODIMENTS **E00)** to **E06),** wherein the protein antibody binding to said 1-AAD-protein, HMGI-C, is
- an antibody (I) comprising a heavy chain variable region with at least 95%, 99% or 100% sequence identity to SEQ ID NO: 1 and a light chain variable region with at least 95%, 99% or 100% sequence identity to SEQ ID NO: 6; OR
- an antibody (II) comprising a heavy chain variable region with the amino acid sequence of SEQ ID NO: 11 or with at least 95% or 99% sequence identity to SEQ ID NO: 11 and a light chain variable region with the amino acid sequence of SEQ ID NO: 16 or with at least 95% or 99% sequence identity to SEQ ID NO: 16;
   OR
- an antibody (III) comprising a heavy chain variable region with the amino acid sequence of SEQ ID NO: 11 or with at least 95% or 99% sequence identity to SEQ ID NO: 21 and a light chain variable region with the amino acid sequence of SEQ ID NO: 16 or with at least 95% or 99% sequence identity to SEQ ID NO: 26.

**E03S)** The method according to any one of EMBODIMENTS **E00)** to **E06),** wherein the protein antibody binding to said 1-AAD-protein, HMGI-C, comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

### EMBODIMENT F [immunoassay determination of acute aortic dissection (AAD) with comparison step]:

**F01)** A method for the immunoassay determination of acute aortic dissection (AAD) in a patient, said method comprising:
a) incubating a bodily fluid sample of the patient with
   - at least one antibody to a first protein released during an event leading to an acute aortic dissection (1-AAD-protein) and
   - a binding partner B for either 1-AAD-protein or the antibody

   wherein either the antibody or the binding partner B is conjugated to a detection agent,
   to form an immunological complex containing the detection agent;
b) isolating the immunological complex from the remaining sample; and
c) determining the detection agent either in the isolated complex or in the remaining sample as an indicator of the 1-AAD-protein in the sample;
d) comparing the said value of said 1-AAD-protein with a known reference of the respective bodily fluid of a human without acute aortic dissection;
   thereby determining the occurrence of AAD in the patient,
   wherein the 1-AAD-protein is HMGI-C.

In general, it cannot be overestimated that it is a real breakthrough using HMGI-C as the biomarker for determining or identifying AAD and this does fulfill a long-standing need. Even though Belge et al. mention HMGA2/HMGI-C in the context of AAD, the article is focused on tissue and the expression levels seen. The importance of the finding that HMGA2/HMGI-C is released into the blood by the AAD event and the finding that this marker can be detected there (in the blood) as such an indicator is not disclosed by Belge. This is extremely important for the cardiologist and for emergency treatment, given the lethality of AAD and the need for immediate action in this - often overlooked - condition. As said above, there is currently no easy way for identifying AAD in a cardiac emergency situation. Just the fact that CN 105 259 353 A mentions ST2 as a (potential) biomarker in the blood over 8 years ago does not indicate such a breakthrough and the applicant/inventor is not aware of finding this marker in any wider use. And - as said above - Belge does not mention HMGA2/HMGI-C as a biomarker in the blood.

**F02)** The method according to EMBODIMENT **F01),** wherein said incubating step a) is done at an alkaline pH.

**F03)** The method according to EMBODIMENT **F02),** wherein the alkaline pH is pH ≥ 7.5, preferably between 8.0 and 10.0, more preferably between 8.0 and 9.5, between 8.5 and 9.5.

**F04)** The method according to EMBODIMENTS **F01)** to **F03),** wherein the detection agent is colloidal metallic gold.

As can be seen in Example 5 and exemplified there with 3 protein antibodies binding to HMGI-C, it is surprisingly important to install an alkaline pH to achieve a successful incubation for determining AAD by detection of HMGI-C in a bodily fluid, like blood.

In the line of this, it is preferred if the method of EMBODIMENT F the conjugation step is done at an alkaline pH between 8.0 and 10.0, or between 8.5 and 9.5 and the detection agent is colloidal metallic gold.

**F05)** The method according to any one of EMBODIMENTS **F01)** to **F04)** wherein if the method is by its comparison step indicative for the presence of acute aortic dissection (AAD) in said patient, this is triggering surgical treatment of the ascending aorta in said patient.

**F06)** The method according to any one of EMBODIMENTS **F01)** to **F05),** wherein the sample is blood, especially full blood, blood plasma or blood serum.

**F07)** The method according to any one of EMBODIMENTS **F01)** to **F06),** wherein the protein antibody binding to said 1-AAD-protein, HMGI-C, is
- an antibody (I) comprising a heavy chain variable region with at least 95%, 99% or 100% sequence identity to SEQ ID NO: 1 and a light chain variable region with at least 95%, 99% or 100% sequence identity to SEQ ID NO: 6;
   OR
- an antibody (II) comprising a heavy chain variable region with the amino acid sequence of SEQ ID NO: 11 or with at least 95% or 99% sequence identity to SEQ ID NO: 11 and a light chain variable region with the amino acid sequence of SEQ ID NO: 16 or with at least 95% or 99% sequence identity to SEQ ID NO: 16;
   OR
- an antibody (III) comprising a heavy chain variable region with the amino acid sequence of SEQ ID NO: 11 or with at least 95% or 99% sequence identity to SEQ ID NO: 21 and a light chain variable region with the amino acid sequence of SEQ ID NO: 16 or with at least 95% or 99% sequence identity to SEQ ID NO: 26.

**F08)** The method according to any one of EMBODIMENTS **F01)** to **F06),** wherein the protein antibody binding to said 1-AAD-protein, HMGI-C, comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

### EMBODIMENT G [Detection of acute aortic dissection (AAD) with comparison step]:

**G01)** A method for the detection of acute aortic dissection (AAD) by detecting HMGI-C in a sample of bodily fluid of a subject, the method comprising:
1) contacting the sample with a protein antibody binding to HMGI-C and thus able to form HMGI-C/Antibody-conjugates;
2) detecting the presence and quantifying the amount of said HMGI-C/Antibody-conjugate in the sample following step 1); and
3) comparing the value obtained in step 3) with a known reference.

**G02)** The method according to EMBODIMENT **G01),** wherein the bodily fluid is blood, especially is full blood, blood plasma and/or blood serum.

**G03)** The method according to EMBODIMENTS **G01)** and **G02),** wherein said contactting step a) is done at an alkaline pH.

**G04)** The method according to EMBODIMENT **G03),** wherein the alkaline pH is pH ≥ 7.5, preferably between 8.0 and 10.0, more preferably between 8.0 and 9.5, between 8.5 and 9.5.

**G05)** The method according to EMBODIMENTS **G01)** to **G04),** wherein the detection agent is colloidal metallic gold.

As can be seen in Example 5 and exemplified there with 3 protein antibodies binding to HMGI-C, it is surprisingly important to install an alkaline pH to achieve a successful incubation for determining AAD by detection of HMGI-C in a bodily fluid, like blood.

In the line of this, it is preferred if the method of EMBODIMENT **G** the contacting step is done at an alkaline pH between 8.0 and 10.0, or between 8.5 and 9.5 and the detection agent is colloidal metallic gold.

**G06)** The method according to any one of EMBODIMENTS **G01)** to **G04)** wherein if the method is by its comparison step indicative for the presence of acute aortic dissection (AAD) in said patient, this is triggering surgical treatment of the ascending aorta in said patient.

**G07)** The method according to any one of EMBODIMENTS **G01)** to **G06),** wherein the protein antibody binding to HMGI-C is
- an antibody (I) comprising a heavy chain variable region with at least 95%, 99% or 100% sequence identity to SEQ ID NO: 1 and a light chain variable region with at least 95%, 99% or 100% sequence identity to SEQ ID NO: 6;
   OR
- an antibody (II) comprising a heavy chain variable region with the amino acid sequence of SEQ ID NO: 11 or with at least 95% or 99% sequence identity to SEQ ID NO: 11 and a light chain variable region with the amino acid sequence of SEQ ID NO: 16 or with at least 95% or 99% sequence identity to SEQ ID NO: 16;
   OR
- an antibody (III) comprising a heavy chain variable region with the amino acid sequence of SEQ ID NO: 11 or with at least 95% or 99% sequence identity to SEQ ID NO: 21 and a light chain variable region with the amino acid sequence of SEQ ID NO: 16 or with at least 95% or 99% sequence identity to SEQ ID NO: 26.

**G08)** The method according to any one of EMBODIMENTS **G01)** to **G06),** wherein the protein antibody binding to HMGI-C comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

### EMBODIMENT H [Lateral Flow assay HMGI-C + D-Dimer + Troponin (or Tnl)]:

**H01)** A lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein with that protein antibody having been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,

wherein the 1-AAD-protein is HMGI-C and thus the lateral flow device comprises said protein antibody binding to HMGI-C (said 1-AAD-protein);
wherein the lateral flow device further comprises one further protein antibody binding to one further protein potentially also released during said event leading to an acute aortic dissection (2-AAD-protein), wherein the 2-AAD-protein is D-Dimer; and
wherein the lateral flow device further comprises a MI-protein antibody binding to a protein indicative for acute myocardial infarction (MI-protein) wherein the MI-protein is troponin, or especially troponin I.

**H02)** The lateral flow device according to EMBODIMENT **H01),** wherein the detection agent conjugated to the antibody to HMGI-C is colloidal metallic gold.

**H03)** The lateral flow device according to EMBODIMENTS **H01)** or **H02),** wherein the protein antibody binding to said 1-AAD-protein, HMGI-C, is
- an antibody (I) comprising a heavy chain variable region with at least 95%, 99% or 100% sequence identity to SEQ ID NO: 1 and a light chain variable region with at least 95%, 99% or 100% sequence identity to SEQ ID NO: 6;
   OR
- an antibody (II) comprising a heavy chain variable region with the amino acid sequence of SEQ ID NO: 11 or with at least 95% or 99% sequence identity to SEQ ID NO: 11 and a light chain variable region with the amino acid sequence of SEQ ID NO: 16 or with at least 95% or 99% sequence identity to SEQ ID NO: 16;
   OR
- an antibody (III) comprising a heavy chain variable region with the amino acid sequence of SEQ ID NO: 11 or with at least 95% or 99% sequence identity to SEQ ID NO: 21 and a light chain variable region with the amino acid sequence of SEQ ID NO: 16 or with at least 95% or 99% sequence identity to SEQ ID NO: 26.

**H04)** The lateral flow device according to EMBODIMENTS **H01)** or **H02),** wherein the protein antibody binding to said 1-AAD-protein, HMGI-C, comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

**H05)** The lateral flow device according to any one of EMBODIMENTS **H01)** to **H04),** wherein the acute aortic dissection (AAD) is acute aortic syndrome which comprises aortic dissection, including acute type A aortic dissection, and/or one or more of the following aortic dissections/acute aortic syndroms:
- Penetrating Aortic Ulcer and Intramural Hematoma
- Thoracic Aortic Aneurysm
- Chronic Dissection-Indications with Treatment with Branched and Fenestrated Stent-grafts
- Different Therapeutic Modalities for Aortic Arch Dissection Combined with Kommerell's Diverticulum
- Secondary Interventions After Endovascular Repair of Aortic Dissections
- False Aneurysms Complicating Internal Carotid Artery Dissection
- Established Indications for the Invasive Treatment of a Thoracic Aortic Aneurysm
- Aortic Arch Dissection: A Controversy of Classification
- Treatment Algorithms for Patients with (Sub)acute Type B Aortic Dissections
- Descending Thoracic Aortic Dissections
- Differences in Aortopathy in Patients with a Bicuspid Aortic Valve with or Without Aortic Coarctation
- Angina Chest pain
- Dyspnea Accompanied by Hypertension Crisis
- Sudden Coma or
- Shock of Unknown Etiology.

**H06)** The lateral flow device according to any one of EMBODIMENTS **H01)** to **H05),** wherein the lateral flow device according to any one of the proceeding claims, wherein the conjugate-binding protein is an FC-binding protein, preferably wherein the FC-binding protein is Protein G, Protein A or a Protein A/G fusion protein.

**H07)** The lateral flow device according to any one of EMBODIMENTS **H01)** to **H06),** wherein the lateral flow device according to any one of the preceding claims, further comprising a control line located substantially perpendicular to the direction of flow of the sample along the strip, preferably wherein the conjugate pad further comprises an immunoglobulin conjugated to a detection agent so that in operation the sample flows from the sample pad to the conjugate pad and mobilizes the immunoglobulin conjugate which passes over the detection band without reactivity and crosses the control line, preferably wherein deposited at the control line is an antibody capable of binding to the mobilized immunoglobin conjugate as it crosses the control line, with optionally said binding at the control line being indicated by a visible color change; even more preferably wherein the immunoglobulin in the conjugate is from an animal species other than the mammal species from which the sample of bodily fluid is derived.

**H08)** The lateral flow device according to any one of EMBODIMENTS **H01)** to **H07),** wherein the troponin is troponin T (TnT) or troponin I (Tnl), especially is troponin I (Tnl).

**H09)** The lateral flow device according to any one of EMBODIMENTS **H01)** to **H08),** wherein the lateral flow device further comprises one further protein antibody binding to one further protein potentially also released during said event leading to an acute aortic dissection (2-AAD-protein), wherein the 2-AAD-protein is creatinine phosphokinase (CK) or preferably the creatinine phosphokinase MB isozyme (CK-MB).

### EMBODIMENT I [A Device for the detection of HMGI-C]:

**I01)** A device for the detection of acute aortic dissection in a sample of bodily fluid of a mammal, the device comprising:
a) a protein antibody binding to the antigen HMGI-C (1-AAD-protein) and
b) a detection unit able to identify the presence of a conjugate of HMGI-C and the protein antibody binding to the antigen HMGI-C.

In one important embodiment of EMBODIMENT I), the device is not a lateral flow device.

In one other important embodiment of EMBODIMENT I), the device is not for the detection of cancer.

In another important embodiment of EMBODIMENT I), the device is not a lateral flow device and is not for the detection of cancer.

**I02**) The device according to EMBODIMENT **I01)**, wherein the protein antibody binding to the antigen HMGI-C has been conjugated to a detection agent.

**I03**) The device according to EMBODIMENT **I01)**, wherein the detection agent conjugated to the antibody is selected from the group comprising metallic nanoparticles or nanoshells, non-metallic nanoparticles or nanoshells, enzymes, colloidal gold and fluorescent molecules, preferably wherein the detection agent conjugated to the antibodies comprises nanoparticles or nanoshells of metallic gold or colloidal gold.

**I04**) The device according to any one of the EMBODIMENTS **I01)** to **I03),** wherein the protein antibody binding to the antigen HMGI-C device is provided in the device at an alkaline pH.

**I05**) The device according to EMBODIMENT **I04**), wherein the alkaline pH is ≥ 7.5, preferably is between 8.0 and 10.0, more preferably is between 8.0 and 9.5 or between 8.5 and 9.5.

**I06**) The device according to any one of the EMBODIMENTS **I01)** to **I05),** wherein the alkaline pH is provided by choice of the device material and/or a chemical buffer comprised in the lateral flow device.

**I07**) The device according to any one of the EMBODIMENTS **I01)** to **I06),** wherein the device comprises an alkaline buffer material, preferably in the part of the device comprising the protein antibody binding to the antigen HMGI-C.

**I08**) The device according to any one of the EMBODIMENTS **I07)**, wherein the buffer is selected to show a pH ≥ 7.5, preferably a pH between 8.0 and 10.0, more preferably a pH between 8.0 and 9.5, or a pH between 8.5 and 9.5, if contacted with a fluid, preferably with a bodily fluid, most preferably with blood, like full blood, blood plasma or blood serum.

**I09**) The device according to any one of the EMBODIMENTS **I01)** to **I08),** wherein said protein antibody binding to HMGI-C is
- an antibody (I) comprising a heavy chain variable region with the amino acid sequence of SEQ ID NO: 1 or with at least 95%, 99% or 100% sequence identity to SEQ ID NO: 1, preferably with the amino acid sequence of SEQ ID NO: 1, and a light chain variable region with the amino acid sequence of SEQ ID NO: 11 or with at least 95%, 99% or 100% sequence identity to SEQ ID NO: 6, preferably with the amino acid sequence of SEQ ID NO: 6;
   OR
- an antibody (II) comprising a heavy chain variable region with the amino acid sequence of SEQ ID NO: 11 or with at least 95% or 99% sequence identity to SEQ ID NO: 11, preferably with the amino acid sequence of SEQ ID NO: 11, and a light chain variable region with the amino acid sequence of SEQ ID NO: 16 or with at least 95% or 99% sequence identity to SEQ ID NO: 16, preferably with the amino acid sequence of SEQ ID NO: 16;
   OR
- an antibody (III) comprising a heavy chain variable region with the amino acid sequence of SEQ ID NO: 11 or with at least 95% or 99% sequence identity to SEQ ID NO: 21, preferably with the amino acid sequence of SEQ ID NO: 21, and a light chain variable region with the amino acid sequence of SEQ ID NO: 16 or with at least 95% or 99% sequence identity to SEQ ID NO: 26, preferably with the amino acid sequence of SEQ ID NO: 26.

**I10)** The lateral flow device according to any one of the EMBODIMENTS **I01)** to **I08**), wherein the protein antibody binding to said 1-AAD-protein, HMGI-C, comprises
- in the heavy chain variable region amino acid sequence CDR1 of SEQ ID NO.2, amino acid sequence CDR2 of SEQ ID NO.3, and amino acid sequence CDR3 of SEQ ID NO.4 and
- in the light chain variable region ONE (VL-1) amino acid sequence CDR1 of SEQ ID NO. 7, amino acid sequence CDR2 of SEQ ID NO. 8 and amino acid sequence CDR3 of SEQ ID NO. 9;
   OR
- in the heavy chain variable region amino acid sequence CDR1 of SEQ ID NO.12, amino acid sequence CDR2 of SEQ ID NO. 13, and amino acid sequence CDR3 of SEQ ID NO. 14 and
- in the light chain variable region amino acid sequence CDR1 of SEQ ID NO. 17, amino acid sequence CDR2 of SEQ ID NO. 18 and amino acid sequence CDR3 of SEQ ID NO. 19;
   OR
- in the heavy chain variable region amino acid sequence CDR1 of SEQ ID NO.22, amino acid sequence CDR2 of SEQ ID NO.23, and amino acid sequence CDR3 of SEQ ID NO.24 and
- in the light chain variable region amino acid sequence CDR1 of SEQ ID NO. 27, amino acid sequence CDR2 of SEQ ID NO. 28 and amino acid sequence CDR3 of SEQ ID NO. 29.

The invention is further described with reference to the following examples. It will be appreciated that the invention as claimed is not intended to be limited in any way by these examples.

### EXAMPLES

### EXAMPLE 1) Development of monoclonal antibodies against HMGI-C derived peptides

It was a

### A. Antibody Development

### 1. Animal Immunization

**Species:** Mouse
**Number of animals: 5**
**Antigen:** Peptide mix
**Immunization protocol:** Standard 45 days, 4 injections

| **Peptide/ Epitope** | **1** | **2** | **3** |
|---|---|---|---|
| **Sequence** | C-QKRGRGRPRKQQQE | C-IFSRDRVSP | C-RGRPRKWAGVQWYNLGSLQ |
| **MW** | **1855.12** | **1179.35** | **2375.73** |
| **Purity** | **>90%** | **>90%** | **>90%** |

### ELISA titer results after the last boost (before fusion)

NB: Negative = pre-immune serum: 1:2000 > 0.2; Standard ELISA without optimization for the respective antigen

### Coating: BSA-C-QKRGRGRPRKQQQE (5µg/ml)

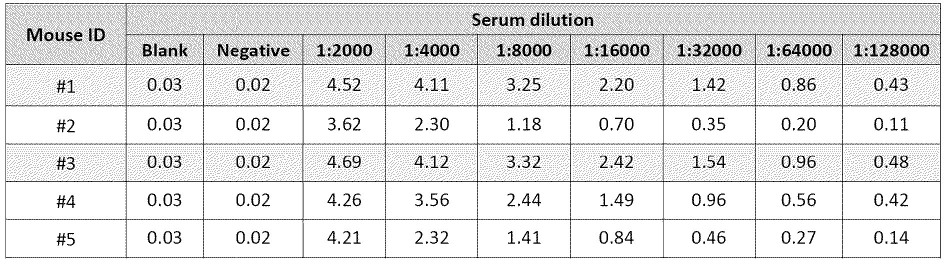

### Coating: BSA-C-RGRPRKWAGVQWYNLGSLQ (5µg/ml)

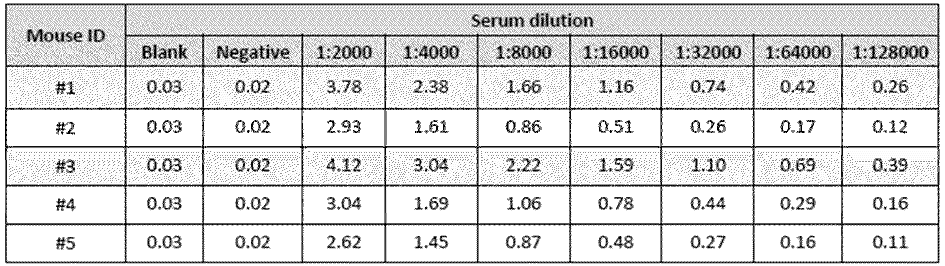

### Coating: IFSRDRVSP-C-BSA (5µg/ml)

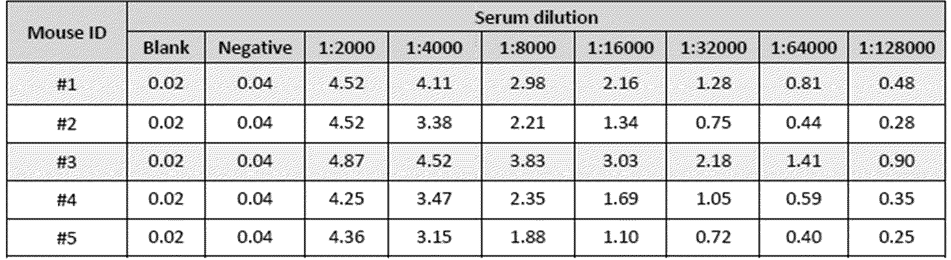

### Animals chosen for the fusion: MOUSE #1 & MOUSE #3

### 2. Fusion * clone screening/selection

### Screening strategy:

- Screen against each of the three peptides individually
- Application: ELISA
- Additional positive screening against protein/peptide different from antigen: No
- Additional negative screening; No

### Clone selection:

- **Clones selected after fusion:** 3 clones
   ∘ Clone 4 with good specific binding against peptide QKRGRGRPRKQQQE
   ∘ Clone 43 with good specific binding against peptide IFSRDRVSP
   ∘ No clone was originally identified with specific binding against peptide RGRPRKWAGVQWYNLGSLQ. Mouse #1 was given booster shots specifically with peptide RGRPRKWAGVQWYNLGSLQ to obtain clones against this peptide. Clone #141 was later identified as specific for this peptide
- 2-3 rounds of sub-cloning performed

### Final clones obtained:

### Final clone: 4-H2-2-G6-E12 (PCC-1)

with good specific binding against peptide QKRGRGRPRKQQQE

lsotype:lgG1
- 3 rounds of sub-cloning

### Final clone: 43-3-C5-A6 (PCC-2)

with good specific binding against peptide IFSRDRVSP

Isotype: lgG2b
- 2 rounds of sub-cloning

### Final clone: 141-3-F2-H4 (PCC-3)

- with good binding to **RGRPRK**WAGVQWYNLGSLQ but also displays binding to QKRG**RGRPRK**QQQE (peptide 1 above) due to the overlap in 6 shared amino acids (see SEQ ID NO: 35 and underlined)
   Isotype: lgG2b
- 2 rounds of sub-cloning

### ELISA results of final clones (supernatants)

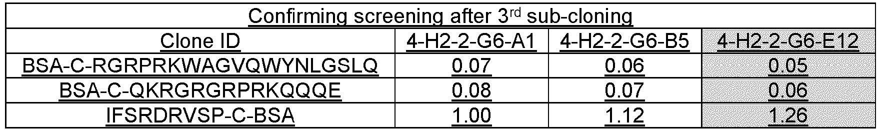

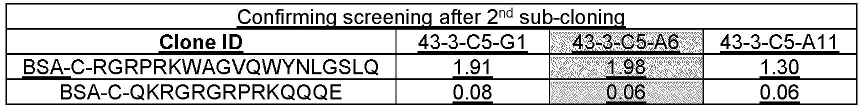

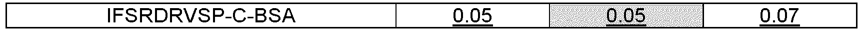

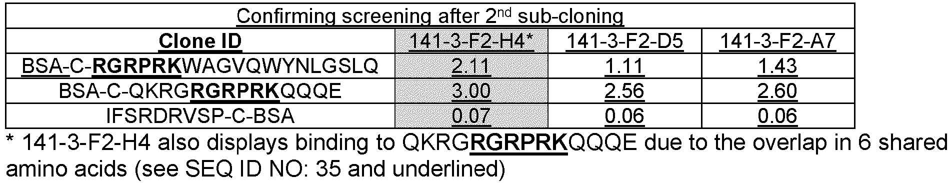

### PURIFICATION SHOWN IN SDS PAGE:

**Fig. 14A****)** shows an SDS PAGE (QC) and shows the purified antibody production from the final clones 4-H2-2-G6-E12 and 43-3-C5-A6 with:
- lane no. 1 showing antibody-43-3-C5-A6 (PCC-2) (2µg, 10% reduce SDS-PAGE)
- lane no. 2 showing antibody-4-H2-2-G6-E12 (PCC-1) (2µg, 10% reduce SDS-PAGE)
- lane no. 3 showing antibody-43-3-C5-A6 (PCC-2) (2µg, 10% non-reduce SDS-PAGE)
- lane no. 4 showing antibody-4-H2-2-G6-E12 (PCC-1) (2µg, 10% non-reduce SDS-PAGE).

**Fig. 14B****)** shows an SDS PAGE (QC) and shows the purified antibody production from the final clone 141-3-F2-H4 with:
- lane no. 1 showing antibody-141-3-F2-H4 (PCC-3) (2µg, 10% reduce SDS-PAGE)
- lane no. 2 showing antibody-141-3-F2-H4 (PCC-3) (2µg, 10% non-reduce SDS-PAGE)

### EXAMPLE 2) Sequencing of full-length HC and LC genes of hybridomas

Full-length HC and LC genes of hybridomas were sequenced. As already laid out in Example 1), these hybrodomas (clones) were named 4-H2-2-G6-E12 (abbreviated hereinafter "4-H2"), 43-3-C5-A6 (abbreviated hereinafter "43-3") and 141-3-F2-H4 (abbreviated hereinafter "141-3"). Hybrodoma 4-H2 was designed against the peptide QKRGRGRPRKQQQE. This peptide is the Epitope 1 (SEQ ID NO. 32) of HMGI-C. Hybrodoma 43-3 was designed against peptide IFSRDRVSP. This peptide is the Epitope 2 (SEQ ID NO. 33) of human HMGI-C. Hybrodoma 141-3 was designed against peptide RGRPRKWAGVQWYNLGSLQ. This peptide is the Epitope 3 (SEQ ID NO. 34) of human HMGI-C.

### Overview

Total RNA was extracted from the hybridoma cells and cDNA subsequently synthesized. Antibody full length genes were then amplified by isotype-specific PCR, subcloned into a standard cloning vector separately and sequenced.

### Materials

- Hybridoma clones, commercial
- TaKaRa MiniBEST Universal RNA Extraction Kit (Takara)
- PrimeScript RT reagent Kit with gDNA Eraser (Takara)
- TA-cloning Kit (Takara)

### Methods

Total RNA was extracted separately from the hybridoma cell line, cDNA was then synthetized by reverse transcription using oligo-dT primers and HC/LC were finally amplified by PCR. HC/LC fragments, respectively amplified by IgG degenerate primers and kappa specific primers, could be observed by gel electrophoresis. The PCR products were then sub-cloned into a standard vector, followed by clone picking and validation by PCR and finally sequencing of positive clones. Sequences were analyzed via Vbase2 and IMGT/V-QUEST.

The discovered sequences are found in the Sequence Listing below.

| **Sequence** | **Number of Clones sequenced** | **Clones with <99% sequence Identity** |
|---|---|---|
| **4-H2-2-G6-E12-HC** | 5 | 100% |
| **4-H2-2-G6-E12-LC** | 5 | 100% |
| **43-3-C5-A6-HC** | 5 | 100% |
| **43-3-C5-A6-LC** | 5 | 100% |
| **141-3-F2-H4-HC** | 5 | 100% |
| **141-3-F2-H4-LC** | 5 | 100% |

### EXAMPLE 3) Further HPLC Analysis of Antibody "43-3" (PCC-2), Antibody "4-H2" (PCC-1) and Antibody "141-3" (PCC-3)

### HPLC Analyses of the antibodies PCC1-3

### Materials and Methods:

### Instruments:

An Agilent 1260 Infinity II HPLC system with the following configuration was used:
- Agilent 1260 Infinity II quaternary pump (G7111B)
- Agilent 1260 Infinity II multisampler (G7167A)
- Agilent 1260 Infinity II multicolumn thermostat (G7116A)
- Agilent 1260 Infinity II variable wavelength detector (G7115A)

### Column:

Agilent Bio SEC-3, 300 Å, 3 µm, 7,8 × 300 mm (p/n 1590-2511)

### Chemicals:

| | | |
|---|---|---|
| NaH₂PO₄.2H₂O (FW 156.01) | 9,36 g/l (0.30 moles) | **mixed with** |
| Na₂HPO₄ anhyd. (FW 141.96) | 12,78 g/l (0.45 moles) | |

Dissolved in 1 L UHP water (e.g. Milli-Q)
→ 1I Puffer with 150 mM, pH 7,0

### Results:

All three antibodies showed a peak at all three measured wavelengths after a retention time of 5 - 6 min. These observations lead to the conclusion that all three antibodies have a very similar size. However, PCC1 is more shifted towards a retention time of 6 min suggesting a smaller size than PCC2 and PCC3. The response in mAU differed slightly between the samples of PCC1, PCC2 and PCC3. PCC1 showed a response of 2,0 - 2,2 mAU, PCC2 a response of 1,6 mAU and PCC3 a response of 1,9 mAU. The different responses in mAU are a result of different sample concentrations applied to the HPLC and/or different running behavior through the matrix of the HPLC column by the different antibodies.

The results are shown in Fig. 15).

HPLC analyses of the antibodies PCC1, PCC2 and PCC3

(A-E) 17 µg/ml of the Antibody PCC1, (F-J) PCC2 and (K-O) PCC3 were analyzed by HPLC. For the analyses by HPLC samples were diluted in NaPO4 Buffer 150 mM pH 7. Antibodies were analyzed at wavelength 220.4 nm, 280,4 nm and 158,4 nm. n = 5 technical replicates

### EXAMPLE 4) HPLC Analysis of Antibody "43-3" (PCC-2), Antibody "4-H2" (PCC-1) and Antibody "141-3" (PCC-3)

### HPLC Analyses of the antibodies PCC1-3

### Materials and Methods:

### Instruments:

An Agilent 1260 Infinity II HPLC system with the following configuration was used:
- Agilent 1260 Infinity II quaternary pump (G7111B)
- Agilent 1260 Infinity II multisampler (G7167A)
- Agilent 1260 Infinity II multicolumn thermostat (G7116A)
- Agilent 1260 Infinity II variable wavelength detector (G7115A)

### Column:

Agilent Bio SEC-3, 300 Å, 3 µm, 7,8 × 300 mm (p/n 1590-2511)

### Chemicals:

| | | |
|---|---|---|
| NaH₂PO₄.2H₂O (FW 156.01) | 9,36 g/l (0.30 moles) | **mixed with** |
| Na₂HPO₄ anhyd. (FW 141.96) | 12,78 g/l (0.45 moles) | |

Dissolved in 1 L UHP water (e.g. Milli-Q)
→ 1I Puffer with 150 mM, pH 7,0

### Results:

All three antibodies showed three characteristic peaks at all three measured wavelengths after different retention time. These observations lead to the conclusion that all three antibodies have fragments of different size after denaturation. However, PCC1 is more shifted towards a later retention time suggesting a smaller size of the fragments than PCC2 and PCC3. The response in mAU differed slightly between the samples of PCC1, PCC2 and PCC3. PCC1 and PCC3 showed smaller peaks than PCC2 leading to the assumption that the denaturation for PCC2 was most efficient. The different responses in mAU are a result of different running behavior through the matrix of the HPLC column by the different antibody fragments due to different denaturation efficiency (Fig.16).

The results are shown in Fig. 16).

HPLC analyses of the denatured antibodies PCC1, PCC2 and PCC3. (A) 25 µg/ml of the Antibody PCC1, (B) PCC2 and (C) PCC3 were analyzed by HPLC. For the analyses by HPLC samples were diluted in NaPO₄ Buffer 150 mM pH 7. Antibodies were analyzed at wavelength 220.4 nm, 280,4 nm and 158,4 n

### EXAMPLE 5) Determination of the optimal pH of Antibody "43-3" (PCC-2), Antibody "4-H2" (PCC-1) and Antibody "141-3" (PCC-3) if conjugated

Determination of the optimal pH value for conjugations of the monoclonal antibodies PCC-1, PCC-2 and PCC-3 to 40 nm colloidal golds.

### Execution

The conjugation is carried out in a 2 mM buffer. The pH values considered and the associated buffer substances are: 6.5 (sodium citrate) 7.0; 7.5; 8.0 (sodium phosphate) 8.5; 9.0; 9.5 (sodium tetraborate) A solution with a volume of 60 µl and a concentration of 0.2 mg/ml was prepared from each of the three antibodies using the respective buffer. A dilution of 1 OD of the gold colloid used (manufacturer Abcam, concentration 10 OD) was prepared for each pH value with the appropriate buffer and 1 ml of this dilution was mixed with the 60 µl of the respective antibody in a 2 ml Eppendorff reaction vessel and the resulting conjugation mixture is incubated for 30 min at room temperature. This was followed by the addition of 120 µl of a 10% BSA solution and a further incubation of 15 minutes at room temperature. Afterwards, all conjugations were stored overnight at 4°C and analyzed the next day. For this purpose, an optical assessment was first carried out and then, for all approaches that produced a conjugate, a spectrophotometric determination of the peak wavelength of the conjugate as well as the absorption in comparison to the peak wavelength and the absorption at this wavelength of the colloid used before protein was added.

### Results and discussion

A stable conjugate should have a red color after overnight storage at 4°C. An aggregated or disintegrated conjugate results in a gray or water-clear solution with a black precipitate in the vessel. The appearance of the conjugates of the three antibodies at the different pH values tested is shown in Fig. 17).

Fig. 17): Conjugates with the 3 tested antibodies, prepared at the indicated pH values between 6.5 and 9.5 after storage at 4°C overnight.

The tested antibodies have different pH dependencies in their conjugate stability. While antibody PCC-1 appears to be conjugable over a very wide pH range (7.0 - 9.5), antibody PCC-2 can only be conjugated at two pH values (9.0 and 9.5). Antibody PCC-3 can be conjugated at pH - Values between 7.5 and 9.5. For the stable conjugates, the peak wavelengths (λpeak) and the associated absorption of the conjugates were determined in the range between 400 and 700 nm in a spectrophotometer. These were compared with the values obtained for these parameters for the various colloids before conjugation (Table 1).

**Tab1: Comparison of λpeak and absorption of the conjugates at different pH values with the starting colloid before conjugation.**

| **Antibody-No.** | **pH** | **λpeak (nm), Colloid** | **Absorption (rel. Units) Colloid** | **Apeak (nm), Conjugate** | **Absorption (rel. Units) Conjugate** | **Apeak Conjugate λpeak (Colloid) (nm)** | **Absorption Conjugate Absorption (Colloid) (rel. Units)** |
|---|---|---|---|---|---|---|---|
| PCC-1 | 7.0 | 522,6 | 1,1319 | 527,8 | 0,9700 | 5,1 | - 0,1619 |
| | 7.5 | 522,8 | 1,1362 | 527,3 | 1,0644 | 4,5 | - 0,0718 |
| | 8.0 | 522,4 | 1,1364 | 526,9 | 1,1189 | 4,5 | - 0,0175 |
| | 8.5 | 522,1 | 0,9497 | 526,3 | 0,9297 | 4,2 | - 0,0200 |
| | 9.0 | 522,5 | 1,1091 | 526,5 | 1,0715 | 4,1 | - 0,0376 |
| | 9.5 | 522,7 | 1,1301 | 526,1 | 1,0606 | 3,4 | - 0,0695 |
| PCC-2 | 9.0 | 522,5 | 1,1091 | 528,0 | 0,9495 | 5,5 | - 0,1596 |
| | 9.5 | 522,7 | 1,1301 | 527,1 | 1,0413 | 4,4 | - 0,0888 |
| PCC-3 | 8.0 | 522,4 | 1,1364 | 527,2 | 1,0675 | 4,8 | - 0,0689 |
| | 8.5 | 522,1 | 0,9497 | 526,7 | 0,9398 | 4,6 | - 0,0099 |
| | 9.0 | 522,5 | 1,1091 | 526,4 | 1,1000 | 3,9 | - 0,0091 |
| | 9.5 | 522,7 | 1,1301 | 526,6 | 1,0819 | 3,9 | - 0,0482 |

In the case of gold conjugations, a shift in the peak wavelength usually occurs when measuring spectra in the wavelength range between 400 and 700 nm. For antibody conjugations, this should not be larger than 6 nm; larger shifts indicate the beginning of aggregation, which makes a conjugate unusable. Larger shifts were not observed for any of the conjugates in Table 1. At the same time, the loss of maximum absorption should be as small as possible compared to the starting colloid. The losses consist of dilution effects due to the addition of the antibody solution and the BSA as well as losses of colloid. This is compensated for by the attachment of proteins to the colloids. The preferred pH values for successful conjugation are 8.5 for antibody PCC-1, 9.5 for antibody PCC-2 and 9.0 for antibody PCC-3.

In summary, it can be stated that all three antibodies are conjugable to gold and could therefore in principle be used in both a sandwich and a competitive test.

### Publications:

1. Isselbacher EM. Disease of the aorta. Heart Disease, 6th edn. Braunwald E, Zipes DP, Libby P, Eds. W.B. Saunders Company, Philadelphia, 2001;1422-56.
2. Nienaber CA, Fattori R, Mehta RH, Richartz BM, Evangelista A, Petzsch M, et al. The International Registry of Acute Aortic Dissection (IRAD). Gender-Related differences in acute aortic dissection. Circulation. 2003;109:3014-21.
3. Pearson GD, Devereux R, Loeys B, Maslen C, Milewicz D, Pyeritz R, Ramirez F, et al. Report of the National Heart, Lung, and Blood Insti- tute and National Marfan Foundation Working Group on research in Marfan syndrome and related disorders. Circulation. 2008;118: 785-91.
4. Parapia LA, Jackson C. Ehlers-Danlos syndrome-a historical review. Br J Haematol. 2008;141:32-5.
5. Callewaert B, Malfait F, Loeys B, De Paepe A. Ehlers-Danlos syn- dromes and Marfan syndrome. Best Pract Res Clin Rheumatol. 2008;22:165-89.
6. Roberts CS, Roberts WC. Dissection of the aorta with congenital malformation of the aortic valve. J Am Coll Cardiol. 1991;17: 712-6.
7. Mehta RH, Suzuki T, Hagan PG, et al. International Registry of Acute Aortic Dissection (IRAD). Investigators. Predicting death in patients with acute type a aortic dissection. Circulation. 2002;105: 200-6.
8. Daily PO, Trueblood HW, Stinson EB, Wuerflein RD, Shumway NE. Management of acute aortic dissections. Ann Thorac Surg. 1970;10: 237-47.
9. DeBakey ME, McCollum CH, Crawford ES, Morris GC, Howell J, Noon GP, Lawrie G. Dissection and dissecting aneurysms of the aorta: twenty-year follow-up of five hundred finienthy-seven patients treated surgically. Surgery. 1980;92:1118-34.
10. Anagnostopoulos CE, Prabhakar MJS, Kittle CF. Aortic dissections and dissecting aneurysms. Am J Cardiol. 1972;30:263-73.
11. Estrera AL, Huynh TT, Porat EE, Miller CC 3rd, Smith JJ, Safi HJ. Is acute type A aortic dissection a true surgical emergency? Semin Vasc Surg. 2002;15:75-82.
12. Miller DC. Acute dissection of the aorta: continuing need for earlier diagnosis and treatment. Mod Con Cardiovasc Dis. 1985;54:51-55.
13. Roberts WC. Aortic dissection: anatomy, consequences, and causes. Am Heart J. 1981;101:195-214.
14. König et al. Aggrecan: a new biomarker for acute type A aortic dissection. Sci Rep 11, 10371 (2021). https://doi.org/10.1038/s41598-021-89653-y.
15. Belge et al. Upregulation of the high mobility group AT-hook 2 gene in acute aortic dissection is potentially associated with endothelial-mesenchymal transition. Histol Histopathol. 2011 Aug;26(8):1029-37. doi: 10.14670/HH-26.1029.
16. CN 105 259 353 A (Beijing Inst. of Heart Lung and Bood Vesser Diseases) of 20 January 2016.
17. US2013/217015 A1 (Nicosia Santo V et al. [US]) of 22 August 2013

### SEQUENCE LISTING

**SEQ ID NO: 1**
   Amino acid sequence of the heavy chain variable domain (VH) of the antibody 4-H2 with the CDRs underlined. 
EVQLQQSGPE LVKPGASVRI SCKASGYSFT GHYMHWVKQS PENSLEWIGE INPTTGGITY
NQKFKGKATL TVDKSSITAY MHLKSLTSED SAVYYCTGGG DHFDYWGQGT TLTVSSAKTT
PPSVYPLAPG SAAQTNSMVT LGCLVKGYFP EPVTVTWNSG SLSSGVHTFP AVLQSDLYTL
SSSVTVPSST WPSETVTCNV AHPASSTKVD KKIVPRDCGC KPCICTVPEV SSVFIFPPKP
KDVLTITLTP KVTCVVVDIS KDDPEVQFSW FVDDVEVHTA QTQPREEQFN STFRSVSELP
IMHQDWLNGK EFKCRVNSAA FPAPIEKTIS KTKGRPKAPQ VYTIPPPKEQ MAKDKVSLTC
MITDFFPEDI TVEWQWNGQP AENYKNTQPI MDTDGSYFVY SKLNVQKSNW EAGNTFTCSV
LHEGLHNHHT EKSLSHSPGK
**SEQ ID NO: 2**
   Amino acid sequence of CDR1 of the heavy chain variable domain (VH) of the antibody 4-H2:
   GYSFTGHY
**SEQ ID NO: 3**
   Amino acid sequence of CDR2 of the heavy chain variable domain (VH) of the antibody 4-H2:
   INPTTGGI
**SEQ ID NO: 4**
   Amino acid sequence of **CDR3** of the heavy chain variable domain (VH) of the antibody 4-H2:
   TGGGDHFDY
**SEQ ID NO: 5**
   **DNA** sequence of the heavy chain variable domain (VH) of the antibody 4-H2: 
GAGGTCCAAC TGCAGCAGTC TGGACCTGAG CTGGTGAAGC CTGGGGCTTC AGTGAGGATA
TCCTGCAAGG CTTCTGGTTA CTCATTCACT GGCCACTACA TGCACTGGGT GAAGCAAAGT
CCTGAAAATA GTCTTGAGTG GATTGGAGAG ATTAATCCTA CCACTGGGGG TATTACCTAC
AACCAGAAGT TCAAGGGCAA GGCCACATTA ACTGTAGATA AATCCTCCAT CACAGCCTAC
ATGCACCTCA AGAGCCTGAC ATCTGAAGAC TCTGCAGTCT ATTACTGTAC AGGCGGGGGG
GACCACTTTG ACTACTGGGG CCAAGGCACC ACTCTCACAG TCTCCTCAGC CAAAACGACA
CCCCCATCTG TCTATCCACT GGCCCCTGGA TCTGCTGCCC AAACTAACTC CATGGTGACC
CTGGGATGCC TGGTCAAGGG CTATTTCCCT GAGCCAGTGA CAGTGACCTG GAACTCTGGA
TCCCTGTCCA GCGGTGTGCA CACCTTCCCA GCTGTCCTGC AGTCTGACCT CTACACTCTG
AGCAGCTCAG TGACTGTCCC CTCCAGCACC TGGCCCAGCG AGACCGTCAC CTGCAACGTT
GCCCACCCGG CCAGCAGCAC CAAGGTGGAC AAGAAAATTG TGCCCAGGGA TTGTGGTTGT
AAGCCTTGCA TATGTACAGT CCCAGAAGTA TCATCTGTCT TCATCTTCCC CCCAAAGCCC
AAGGATGTGC TCACCATTAC TCTGACTCCT AAGGTCACGT GTGTTGTGGT AGACATCAGC
AAGGATGATC CCGAGGTCCA GTTCAGCTGG TTTGTAGATG ATGTGGAGGT GCACACAGCT
CAGACGCAAC CCCGGGAGGA GCAGTTCAAC AGCACTTTCC GCTCAGTCAG TGAACTTCCC
ATCATGCACC AGGACTGGCT CAATGGCAAG GAGTTCAAAT GCAGGGTCAA CAGTGCAGCT
TTCCCTGCCC CCATCGAGAA AACCATCTCC AAAACCAAAG GCAGACCGAA GGCTCCACAG
GTGTACACCA TTCCACCTCC CAAGGAGCAG ATGGCCAAGG ATAAAGTCAG TCTGACCTGC
ATGATAACAG ACTTCTTCCC TGAAGACATT ACTGTGGAGT GGCAGTGGAA TGGGCAGCCA
GCGGAGAACT ACAAGAACAC TCAGCCCATC ATGGACACAG ATGGCTCTTA CTTCGTCTAC
AGCAAGCTCA ATGTGCAGAA GAGCAACTGG GAGGCAGGAA ATACTTTCAC CTGCTCTGTG
TTACATGAGG GCCTGCACAA CCACCATACT GAGAAGAGCC TCTCCCACTC TCCTGGTAAA
**SEQ ID NO: 6**
   Amino acid sequence of the light chain variable domain (VL) of the antibody 4-H2 with the CDRs underlined. 
DVSMTQSPLS LPVSLGDQVS ISCRSSQSIL HSNGNTYLEW FLQRPGQSPK
LLIYKVSNRF SGVPDRFSGS GSGTDFTLKI SRVEAEDLGV YYCFQGSHDP
PTFGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC
**SEQ ID NO: 7**
   Amino acid sequence of **CDR1** of the light chain variable domain (VL) of the antibody 4-H2:
   **QSILHSNGNTY**
**SEQ ID NO: 8**
   Amino acid sequence of **CDR2** of the light chain variable domain (VL) of the antibody 4-H2:
   **KVS**
**SEQ ID NO: 9**
   Amino acid sequence of **CDR3** of the light chain variable domain (VL) of the antibody 4-H2:
   **FQGSHDPPT**
**SEQ ID NO: 10**
   **DNA** sequence of the light chain variable domain (VL) of the antibody 4-H2: 
GATGTTTCGA TGACCCAAAG TCCACTCTCC CTGCCTGTCA GTCTTGGAGA
TCAAGTCTCC ATCTCTTGCA GATCCAGTCA GAGCATTTTA CATAGTAACG
GAAACACCTA TTTAGAATGG TTCCTGCAGA GACCAGGCCA GTCTCCAAAG
CTCCTGATCT ACAAAGTTTC CAACCGATTT TCTGGGGTCC CAGACAGGTT
CAGTGGCAGT GGATCAGGGA CAGATTTCAC ACTCAAGATC AGCAGAGTGG
AGGCTGAGGA TCTGGGAGTT TATTACTGCT TTCAAGGTTC ACATGATCCT
CCGACGTTCG GTGGAGGCAC CAAGCTGGAA ATCAAACGGG CTGATGCTGC
ACCAACTGTA TCCATCTTCC CACCATCCAG TGAGCAGTTA ACATCTGGAG
GTGCCTCAGT CGTGTGCTTC TTGAACAACT TCTACCCCAA AGACATCAAT
GTCAAGTGGA AGATTGATGG CAGTGAACGA CAAAATGGCG TCCTGAACAG
TTGGACTGAT CAGGACAGCA AAGACAGCAC CTACAGCATG AGCAGCACCC
TCACGTTGAC CAAGGACGAG TATGAACGAC ATAACAGCTA TACCTGTGAG
 
GCCACTCACA AGACATCAAC TTCACCCATT GTCAAGAGCT TCAACAGGAA
TGAGTGT
**SEQ ID NO: 11**
   Amino acid sequence of the heavy chain variable domain (VH) of the antibody 43-3 with the CDRs underlined. 
QIQLVQSGPE LKKPGETVKI SCKASGYTFT NYGMNWVKQA PGKDLKWMGW
INTYTGEPTY ADDFKGRFVF SLETSASTAY LQINNLNNKD TATYFCAREG
RLLYFHVWGA GTTVTVSSAK TTPPSVYPLA PGCGDTTGSS VTLGCLVKGY
FPESVTVTWN SGSLSSSVHT FPALLQSGLY TMSSSVTVPS STWPSQTVTC
SVAHPASSTT VDKKLEPSGP ISTINPCPPC KECHKCPAPN LEGGPSVFIF
PPNIKDVLMI SLTPKVTCVV VDVSEDDPDV RISWFVNNVE VHTAQTQTHR
EDYNSTIRVV SALPIQHQDW MSGKEFKCKV NNKDLPSPIE RTISKIKGLV
RAPQVYILPP PAEQLSRKDV SLTCLVVGFN PGDISVEWTS NGHTEENYKD
TAPVLDSDGS YFIYSKLDIK TSKWEKTDSF SCNVRHEGLK NYYLKKTISR SPGK
**SEQ ID NO. 12**
   Amino acid sequence of **CDR1** of the heavy chain variable domain (VH) of the antibody 43-3:
   **GYTFTNYG**
**SEQ ID NO. 13**
   Amino acid sequence of **CDR2** of the heavy chain variable domain (VH) of the antibody 43-3:
   **INTYTGEP**
**SEQ ID NO. 14**
   Amino acid sequence of **CDR3** of the heavy chain variable domain (VH) of the antibody 43-3:
   **AREGRLLYFHV**
**SEQ ID NO. 15**
   **DNA** sequence of the heavy chain variable domain (VH) of the antibody 43-3: 
CAGATCCAGT TGGTGCAGTC TGGACCTGAG CTGAAGAAGC CTGGAGAGAC
AGTCAAGATC TCCTGCAAGG CTTCTGGGTA TACCTTCACA AACTATGGAA
TGAACTGGGT GAAGCAGGCT CCAGGAAAGG ATTTAAAGTG GATGGGCTGG
ATAAACACCT ACACTGGAGA GCCAACATAT GCTGATGACT TCAAGGACG
GTTTGTCTTC TCTTTGGAAA CCTCTGCCAG CACTGCCTAT TTGCAGATCA
ACAACCTCAA CAATAAAGAC ACGGCTACAT ATTTCTGTGC AAGAGAGGGG
AGGCTACTAT ATTTCCATGT CTGGGGCGCA GGGACCACGG TCACCGTCTC
CTCAGCTAAA ACAACACCCC CATCAGTCTA TCCACTGGCC CCTGGGTGTG
GAGATACAAC TGGTTCCTCT GTGACTCTGG GATGCCTGGT CAAGGGCTAC
TTCCCTGAGT CAGTGACTGT GACTTGGAAC TCTGGATCCC TGTCCAGCAG
TGTGCACACC TTCCCAGCTC TCCTGCAGTC TGGACTCTAC ACTATGAGCA
GCTCAGTGAC TGTCCCCTCC AGCACCTGGC CAAGTCAGAC CGTCACCTGC
AGCGTTGCTC ACCCAGCCAG CAGCACCACG GTGGACAAAA AACTTGAGCC
CAGCGGGCCC ATTTCAACAA TCAACCCCTG TCCTCCATGC AAGGAGTGTC
ACAAATGCCC AGCTCCTAAC CTCGAGGGTG GACCATCCGT CTTCATCTTC
CCTCCAAATA TCAAGGATGT ACTCATGATC TCCCTGACAC CCAAGGTCAC
GTGTGTGGTG GTGGATGTGA GCGAGGATGA CCCAGACGTC CGGATCAGCT
GGTTTGTGAA CAACGTGGAA GTACACACAG CTCAGACACA AACCCATAGA
GAGGATTACA ACAGTACTAT CCGGGTGGTC AGTGCCCTCC CCATCCAGCA
CCAGGACTGG ATGAGTGGCA AGGAGTTCAA ATGCAAGGTC AACAACAAAG
ACCTCCCATC ACCCATCGAG AGAACCATCT CAAAAATTAA AGGGCTAGTC
AGAGCTCCAC AAGTATACAT CTTGCCGCCA CCAGCAGAGC AGTTGTCCAG
GAAAGATGTC AGTCTCACTT GCCTGGTCGT GGGCTTCAAC CCTGGAGACA
TCAGTGTGGA GTGGACCAGC AATGGGCATA CTGAGGAGAA CTACAAGGAC
ACCGCACCAG TCCTGGACTC TGACGGTTCT TACTTCATAT ACAGCAAGCT
CGATATAAAA ACAAGCAAGT GGGAGAAAAC AGATTCCTTC TCATGCAACG
TGAGACACGA GGGTCTGAAA AATTACTACC TGAAGAAGAC CATCTCCCGG
TCTCCGGGTA AA
**SEQ ID NO: 16**
   Amino acid sequence of the light chain variable domain (VL) of the antibody 43-3 with the CDRs underlined. 
DVLMTQTPLS LPVSLGDQAS ISCRSSQRIV HSNGNTYLEW YLQKPGQSPK
LLIYKVSNRF SGVPDRFSGS GSGTDFTLKI TRVEAEDLGV YYCFQGSHVP
YTFGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC
**SEQ ID NO: 17**
   Amino acid sequence of **CDR1** of the light chain variable domain (VL) of the antibody 43-3:
   **QRIVHSNGNTY**
**SEQ ID NO: 18**
   Amino acid sequence of **CDR2** of the light chain variable domain (VL) of the antibody 43-3:
   KVS
**SEQ ID NO: 19**
   Amino acid sequence of **CDR3** of the light chain variable domain (VL) of the antibody 43-3:
   FQGSHVPYT
**SEQ ID NO: 20**
   **DNA** sequence of the light chain variable domain (VL) of the antibody 43-3: 
GATGTTTTGA TGACCCAAAC TCCACTCTCC CTGCCTGTCA GTCTTGGAGA
TCAAGCCTCC ATCTCTTGCA GATCTAGTCA GCGCATTGTA CATAGTAATG
GAAACACGTA TTTAGAATGG TACCTGCAGA AACCAGGCCA GTCTCCAAAG
 
CTCCTGATCT ACAAAGTTTC CAACCGATTT TCTGGGGTCC CAGACAGGTT
CAGTGGCAGT GGATCAGGGA CAGATTTCAC ACTCAAGATC ACCAGAGTGG
AGGCTGAGGA TCTGGGAGTT TATTACTGCT TTCAAGGTTC ACATGTTCCG
TACACATTCG GAGGGGGGAC CAAGCTGGAA ATAAAACGGG CTGATGCTGC
ACCAACTGTA TCCATCTTCC CACCATCCAG TGAGCAGTTA ACATCTGGAG
GTGCCTCAGT CGTGTGCTTC TTGAACAACT TCTACCCCAA AGACATCAAT
GTCAAGTGGA AGATTGATGG CAGTGAACGA CAAAATGGCG TCCTGAACAG
TTGGACTGAT CAGGACAGCA AAGACAGCAC CTACAGCATG AGCAGCACCC
TCACGTTGAC CAAGGACGAG TATGAACGAC ATAACAGCTA TACCTGTGAG
GCCACTCACA AGACATCAAC TTCACCCATT GTCAAGAGCT TCAACAGGAA
TGAGTGT
**SEQ ID NO: 21**
   Amino acid sequence of the heavy chain variable domain (VH) of the antibody 141-3 with the CDRs underlined. 
EVKLEQSGPE LKKPGETVKI SCMASGYTFT DYSIHWVRQA PGKGLKWMAW
INTETGETTY ADDFKGRFAF SLETSASTAY LQINNLENED TATYYCARWG
YDYYDGMDYW GQGTSVTVSS AKTTPPSVYP LAPGCGDTTG SSVTLGCLVK
GYFPESVTVT WNSGSLSSSV HTFPALLQSG LYTMSSSVTV PSSTWPSQTV
TCSVAHPASS TTVDKKLEPS GPISTINPCP PCKECHKCPA PNLEGGPSVF
IFPPNIKDVL MISLTPKVTC VVVDVSEDDP DVRISWFVNN VEVHTAQTQT
HREDYNSTIR VVSALPIQHQ DWMSGKEFKC KVNNKDLPSP IERTISKIKG
LVRAPQVYIL PPPAEQLSRK DVSLTCLVVG FNPGDISVEW TSNGHTEENY
KDTAPVLDSD GSYFIYSKLD IKTSKWEKTD SFSCNVRHEG LKNYYLKKTI
SRSPGK
**SEQ ID NO. 22**
   Amino acid sequence of **CDR1** of the heavy chain variable domain (VH) of the antibody 141-3:
   **GYTFTDYS**
**SEQ ID NO. 23**
   Amino acid sequence of **CDR2** of the heavy chain variable domain (VH) of the antibody 141-3:
   **INTETGET**
**SEQ ID NO. 24**
   Amino acid sequence of **CDR3** of the heavy chain variable domain (VH) of the antibody 141-3:
   **ARWGYDYYDGMDY**
**SEQ ID NO. 25**
   DNA sequence of the heavy chain variable domain (VH) of the antibody 141-3: 
GAGGTGAAGC TGGAGCAGTC AGGACCTGAG CTGAAGAAGC CTGGAGAGAC
AGTCAAGATC TCCTGCATGG CTTCTGGTTA CACCTTCACA GACTATTCAA
TTCACTGGGT GAGGCAGGCT CCAGGAAAGG GTTTAAAGTG GATGGCCTGG
ATAAACACTG AGACTGGTGA GACAACATAT GCAGATGACT TCAAGGACG
GTTTGCCTTC TCTTTGGAAA CCTCTGCCAG CACGGCCTAT TTGCAGATCA
ACAACCTCGA AAATGAGGAC ACGGCTACAT ATTACTGTGC TAGGTGGGGG
TATGATTACT ACGATGGTAT GGACTACTGG GGTCAAGGAA CTTCAGTCAC
CGTCTCCTCA GCTAAAACAA CACCCCCATC AGTCTATCCA CTGGCCCCTG
GGTGTGGAGA TACAACTGGT TCCTCTGTGA CTCTGGGATG CCTGGTCAAG
GGCTACTTCC CTGAGTCAGT GACTGTGACT TGGAACTCTG GATCCCTGTC
CAGCAGTGTG CACACCTTCC CAGCTCTCCT GCAGTCTGGA CTCTACACTA
TGAGCAGCTC AGTGACTGTC CCCTCCAGCA CCTGGCCAAG TCAGACCGTC
ACCTGCAGCG TTGCTCACCC AGCCAGCAGC ACCACGGTGG ACAAAAAACT
TGAGCCCAGC GGGCCCATTT CAACAATCAA CCCCTGTCCT CCATGCAAGG
AGTGTCACAA ATGCCCAGCT CCTAACCTCG AGGGTGGACC ATCCGTCTTC
ATCTTCCCTC CAAATATCAA GGATGTACTC ATGATCTCCC TGACACCCAA
GGTCACGTGT GTGGTGGTGG ATGTGAGCGA GGATGACCCA GACGTCCGGA
TCAGCTGGTT TGTGAACAAC GTGGAAGTAC ACACAGCTCA GACACAAACC
CATAGAGAGG ATTACAACAG TACTATCCGG GTGGTCAGTG CCCTCCCCAT
CCAGCACCAG GACTGGATGA GTGGCAAGGA GTTCAAATGC AAGGTCAACA
 
ACAAAGACCT CCCATCACCC ATCGAGAGAA CCATCTCAAA AATTAAAGGG
CTAGTCAGAG CTCCACAAGT ATACATCTTG CCGCCACCAG CAGAGCAGTT
GTCCAGGAAA GATGTCAGTC TCACTTGCCT GGTCGTGGGC TTCAACCCTG
GAGACATCAG TGTGGAGTGG ACCAGCAATG GGCATACTGA GGAGAACTAC
AAGGACACCG CACCAGTCCT GGACTCTGAC GGTTCTTACT TCATATACAG
CAAGCTCGAT ATAAAAACAA GCAAGTGGGA GAAAACAGAT TCCTTCTCAT
GCAACGTGAG ACACGAGGGT CTGAAAAATT ACTACCTGAA GAAGACCATC
TCCCGGTCTC CGGGTAAA
**SEQ ID NO: 26**
   Amino acid sequence of the light chain variable domain (VL) of the antibody 141-3 with the CDRs underlined. 
DVQITQSPSY LAASPGETIT INCRASKRIS KNLAWYQEKP GKTIKLLIYS
GSTLQSGIPS RFSGSGSGRD FTLIISSLEP EDIAMYYCQQ NNEYPLTFGA
GTKLELKRAD AAPTVSIFPP SSEQLTSGGA SVVCFLNNFY PKDINVKWKI
DGSERQNGVL NSWTDQDSKD STYSMSSTLT LTKDEYERHN SYTCEATHKT
STSPIVKSFN RNEC
**SEQ ID NO: 27**
   Amino acid sequence of CDR1 of the light chain variable domain (VL) of the antibody 141-3:
   **KRISKN**
**SEQ ID NO: 28**
   Amino acid sequence of CDR2 of the light chain variable domain (VL) of the antibody 141-3:
   SGS
**SEQ ID NO: 29**
   Amino acid sequence of **CDR3** of the light chain variable domain (VL) of the antibody 141-3:
   **QQNNEYPLT**
**SEQ ID NO: 30**
   DNA sequence of the light chain variable domain (VL) of the antibody 141-3: 
GATGTCCAGA TAACCCAGTC TCCATCTTAT CTTGCTGCAT CTCCTGGAGA AACCATTACT
ATTAATTGCA GGGCAAGTAA GAGAATTAGC AAAAATTTAG CCTGGTATCA GGAGAAACCT
GGGAAAACTA TTAAGCTTCT TATCTACTCT GGATCCACTT TGCAATCTGG AATTCCATCA
AGGTTCAGTG GCAGTGGATC TGGTAGAGAT TTCACTCTCA TCATCAGTAG CCTGGAGCCT
GAAGATATTG CAATGTATTA CTGTCAACAG AATAATGAAT ACCCGCTCAC GTTCGGTGCT
GGGACCAAGC TGGAGCTGAA ACGGGCTGAT GCTGCACCAA CTGTATCCAT CTTCCCACCA
TCCAGTGAGC AGTTAACATC TGGAGGTGCC TCAGTCGTGT GCTTCTTGAA CAACTTCTAC
CCCAAAGACA TCAATGTCAA GTGGAAGATT GATGGCAGTG AACGACAAAA TGGCGTCCTG
AACAGTTGGA CTGATCAGGA CAGCAAAGAC AGCACCTACA GCATGAGCAG CACCCTCACG
TTGACCAAGG ACGAGTATGA ACGACATAAC AGCTATACCT GTGAGGCCAC TCACAAGACA
TCAACTTCAC CCATTGTCAA GAGCTTCAAC AGGAATGAGT GT
**SEQ ID NO. 31:**
   Amino acid sequence of human HMGI-C (High mobility group protein) with Epitope 1, Epitope 3 and Epitope 2 underlined. 
1 MSARGEGAGQ PSTSAQGQPA APAPQKRGRG RPRKQQQEPT GEPSPKRPRG
51 RPKGSKNKSP SKAAQKKAEA TGEKRPRGRP RKWAGVQWYN LGSLQPPPPR
101 FKQFSCLRLL SSWDYRHPPP HPANFCIFSR DRVSPCWPGW SRTPDLR
 Just for declaration purpose, the same amino acid sequence of human HMGI-C (High mobility group protein) with Epitope 1, Epitope 3 and Epitope 2 underlined and the identical part in in Epitope 1 and Epitope 3 highlighted in grey. 
1 MSARGEGAGQ PSTSAQGQPA APAPQKRGRG RPRKQQQEPT GEPSPKRPRG
51 RPKGSKNKSP SKAAQKKAEA TGEKRPRGRP RKWAGVQWYN LGSLQPPPPR
101 FKQFSCLRLL SSWDYRHPPP HPANFCIFSR DRVSPCWPGW SRTPDLR
**SEQ ID NO. 32:**
   Amino acid sequence of Epitope 1 of human HMGI-C (High mobility group protein)
   QKRGRGRPRKQQQE
**SEQ ID NO. 33:**
   Amino acid sequence of Epitope 2 of human HMGI-C (High mobility group protein)
   IFSRDRVSP
**SEQ ID NO. 34:**
   Amino acid sequence of Epitope 3 of human HMGI-C (High mobility group protein)
   RGRPRKWAGVQWYNLGSLQ
**SEQ ID NO. 35:**
   Amino acid sequence of the overlap between Epitope 1 and Epitope 3 of human HMGI-C (High mobility group protein)
   RGRPRK
**SEQ ID NO: 36**
   Amino acid sequence of **FR1** of the heavy chain variable domain (VH) of the antibody 4-H2:
   EVQLQQSGPELVKPGASVRISCKAS
**SEQ ID NO: 37**
   Amino acid sequence of **FR2** of the heavy chain variable domain (VH) of the antibody 4-H2:
   MHWVKQSPENSLEWIGE
**SEQ ID NO: 38**
   Amino acid sequence of **FR3** of the heavy chain variable domain (VH) of the antibody 4-H2:
   TYNQKFKGKATLTVDKSSITAYMHLKSLTSEDSAVYYC
**SEQ ID NO: 39**
   Amino acid sequence of **FR4** of the heavy chain variable domain (VH) of the antibody 4-H2: 
WGQGT TLTVSSAKTT PPSVYPLAPG SAAQTNSMVT LGCLVKGYFP
EPVTVTWNSG SLSSGVHTFP AVLQSDLYTL SSSVTVPSST WPSETVTCNV
AHPASSTKVD KKIVPRDCGC KPCICTVPEV SSVFIFPPKP KDVLTITLTP
KVTCVVVDIS KDDPEVQFSW FVDDVEVHTA QTQPREEQFN STFRSVSELP
IMHQDWLNGK EFKCRVNSAA FPAPIEKTIS KTKGRPKAPQ VYTIPPPKEQ
MAKDKVSLTC MITDFFPEDI TVEWQWNGQP AENYKNTQPI MDTDGSYFVY
SKLNVQKSNW EAGNTFTCSV LHEGLHNHHT EKSLSHSPGK
**SEQ ID NO: 40**
   Amino acid sequence of **FR1** of the light chain variable domain (VL) of the antibody 4-H2:
   DVSMTQSPLSLPVSLGDQVSISCRSS
**SEQ ID NO: 41**
   Amino acid sequence of **FR2** of the light chain variable domain (VL) of the antibody 4-H2:
   LEWFLQRPGQSPKLLIY
**SEQ ID NO: 42**
   Amino acid sequence of **FR3** of the light chain variable domain (VL) of the antibody 4-H2:
   NRFSGVPDRFSGSGSGTDFTLKISRVEAEDLGVYYC
**SEQ ID NO: 43**
   Amino acid sequence of **FR4** of the light chain variable domain (VL) of the antibody 4-H2: 
FGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC
**SEQ ID NO: 44**
   Amino acid sequence of **FR1** of the heavy chain variable domain (VH) of the antibody 43-3:
   QIQLVQSGPELKKPGETVKISCKAS
**SEQ ID NO: 45**
   Amino acid sequence of **FR2** of the heavy chain variable domain (VH) of the antibody 43-3:
   MNWVKQAPGKDLKWMGW
**SEQ ID NO: 46**
   Amino acid sequence of **FR3** of the heavy chain variable domain (VH) of the antibody 43-3:
   YADDFKGRFVFSLETSASTAYLQINNLNNKDTATYFC
**SEQ ID NO: 47**
   Amino acid sequence of **FR4** of the heavy chain variable domain (VH) of the antibody 43-3: 
WGA

GTTVTVSSAK TTPPSVYPLA PGCGDTTGSS VTLGCLVKGY FPESVTVTWN

SGSLSSSVHT FPALLQSGLY TMSSSVTVPS STWPSQTVTC SVAHPASSTT

VDKKLEPSGP ISTINPCPPC KECHKCPAPN LEGGPSVFIF PPNIKDVLMI

SLTPKVTCVV VDVSEDDPDV RISWFVNNVE VHTAQTQTHR EDYNSTIRVV

SALPIQHQDW MSGKEFKCKV NNKDLPSPIE RTISKIKGLV RAPQVYILPP

PAEQLSRKDV SLTCLVVGFN PGDISVEWTS NGHTEENYKD TAPVLDSDGS

YFIYSKLDIK TSKWEKTDSF SCNVRHEGLK NYYLKKTISR SPGK
**SEQ ID NO: 48**
   Amino acid sequence of **FR1** of the light chain variable domain (VL) of the antibody 43-3:
   DVLMTQTPLSLPVSLGDQASISCRSS
**SEQ ID NO: 49**
   Amino acid sequence of **FR2** of the light chain variable domain (VL) of the antibody 43-3:
   LEWYLQKPGQSPKLLIY
**SEQ ID NO: 50**
   Amino acid sequence of **FR3** of the light chain variable domain (VL) of the antibody 43-3:
   NRFSGVPDRFSGSGSGTDFTLKITRVEAEDLGVYYC
**SEQ ID NO: 51**
   Amino acid sequence of **FR4** of the light chain variable domain (VL) of the antibody 43-3: 
FGGGTKLE IKRADAAPTV SIFPPSSEQL TSGGASVVCF LNNFYPKDIN
VKWKIDGSER QNGVLNSWTD QDSKDSTYSM SSTLTLTKDE YERHNSYTCE
ATHKTSTSPI VKSFNRNEC
**SEQ ID NO: 52**
   Amino acid sequence of **FR1** of the heavy chain variable domain (VH) of the antibody 141-3:
   EVKLEQSGPE LKKPGETVKI SCMAS
**SEQ ID NO: 53**
   Amino acid sequence of **FR2** of the heavy chain variable domain (VH) of the antibody 141-3:
   IHWVRQAPGK GLKWMAW
**SEQ ID NO: 54**
   Amino acid sequence of **FR3** of the heavy chain variable domain (VH) of the antibody 141-3:
   TYADDFKGRF AFSLETSAST AYLQINNLEN EDTATYYC
**SEQ ID NO: 55**
   Amino acid sequence of **FR4** of the heavy chain variable domain (VH) of the antibody 141-3: 
W
GQGTSVTVSS AKTTPPSVYP LAPGCGDTTG SSVTLGCLVK GYFPESVTVT
WNSGSLSSSV HTFPALLQSG LYTMSSSVTV PSSTWPSQTV TCSVAHPASS
TTVDKKLEPS GPISTINPCP PCKECHKCPA PNLEGGPSVF IFPPNIKDVL
MISLTPKVTC VVVDVSEDDP DVRISWFVNN VEVHTAQTQT HREDYNSTIR
VVSALPIQHQ DWMSGKEFKC KVNNKDLPSP IERTISKIKG LVRAPQVYIL
PPPAEQLSRK DVSLTCLVVG FNPGDISVEW TSNGHTEENY KDTAPVLDSD
GSYFIYSKLD IKTSKWEKTD SFSCNVRHEG LKNYYLKKTI SRSPGK
**SEQ ID NO: 56**
   Amino acid sequence of **FR1** of the light chain variable domain (VL) of the antibody 141-3:
   DVQITQSPSY LAASPGETIT INCRAS
**SEQ ID NO: 57**
   Amino acid sequence of **FR2** of the light chain variable domain (VL) of the antibody 43-3:
   LAWYQEKPGK TIKLLIY
**SEQ ID NO: 58**
   Amino acid sequence of **FR3** of the light chain variable domain (VL) of the antibody 141-3:
   TLQSGIPSRF SGSGSGRDFT LIISSLEPED IAMYYC
**SEQ ID NO: 59**
   Amino acid sequence of **FR4** of the light chain variable domain (VL) of the antibody 141-3:

FGA GTKLELKRAD AAPTVSIFPP SSEQLTSGGA SVVCFLNNFY PKDINVKWKI
DGSERQNGVL NSWTDQDSKD STYSMSSTLT LTKDEYERHN SYTCEATHKT STSPIVKSFN
RNEC

## Claims

1. A lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
wherein the 1-AAD-protein is HMGI-C; and
wherein the lateral flow device further comprises a MI-protein antibody binding to a protein indicative for acute myocardial infarction (MI-protein) wherein the MI-protein is troponin I.

2. The lateral flow device according to claim 1, wherein the lateral flow device further comprises one or more further protein antibody/ies binding to one or more further protein/s potentially also released during said event leading to an acute aortic dissection (X-AAD-protein/s), wherein the X-AAD-protein/s is/are selected from a group of biomarkers for AAD including aggrecan, smooth muscle myosin heavy chain, soluble aortic elastin or D-dimer, preferably is D-Dimer
or
wherein the X-AAD-proteins are D-Dimer and isozyme CK-MB;
or
wherein the X-AAD-protein is D-Dimer.

3. A lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
wherein the 1-AAD-protein is HMGI-C; and
wherein the lateral flow device is arranged so that if the sample reaches the first conjugate pad, the fluid in said first conjugate pad shows or turns to an alkaline pH.

4. The lateral flow device according to claim 3, wherein the alkaline pH is ≥ 7.5, preferably is between 8.0 and 10.0, more preferably is between 8.0 and 9.5 or between 8.5 and 9.5.

5. The lateral flow device according to claims 3 or 4, wherein the detection agent conjugated to the antibody is colloidal metallic gold.

6. A lateral flow device for the detection of a first protein released during an event leading to an acute aortic dissection (1-AAD-protein) in a sample of bodily fluid of a mammal, the device comprising:
i) a strip formed of a material enabling capillary flow of fluid along a portion of the strip;
ii) a sample pad located proximal to one end of the strip for receiving the sample of the bodily fluid;
iii) a first conjugate pad located in the strip so that in operation the sample flows under capillary action through the strip from the sample pad to the conjugate pad and mobilizes a conjugate comprised in the conjugate pad, the conjugate comprises a protein antibody binding to said 1-AAD-protein that has been conjugated to a detection agent, and
iv) a first detection band comprising a first conjugate-binding protein immobilized within the strip along a band located substantially perpendicular to the direction of flow of the sample along the strip so that when the mobilized 1-AAD-protein antibody conjugate in the sample contacts the first conjugate-binding protein in the first detection band the presence of said 1-AAD-protein in the sample is indicated by a visible color change,
wherein the 1-AAD-protein is HMGI-C; and
wherein the lateral flow device comprises an alkaline chemical buffer.

7. The lateral flow device according to claim 6, wherein the lateral flow device comprises said alkaline chemical buffer in at least one of the first conjugate pad, the strip between the sample pad and the first conjugate pad, or in the sample pad, preferably in the first conjugate pad.

8. The lateral flow device according to claims 6 or 7, wherein the buffer if contacted with the sample of bodily fluid is set to show a pH ≥ 7.5, preferably between 8.0 and 10.0, more preferably between 8.0 and 9.5, between 8.5 and 9.5.

9. The lateral flow device according to any one of claims 6 to 8, wherein the detection agent conjugated to the antibody is colloidal metallic gold.

10. A method for the immunoassay determination of acute aortic dissection (AAD) in a patient, said method comprising:
a) incubating a bodily fluid sample of the patient with
∘ at least one antibody to a first protein released during an event leading to an acute aortic dissection (1-AAD-protein) and
∘ a binding partner B for either 1-AAD-protein or the antibody
wherein either the antibody or the binding partner B is conjugated to a detection agent,
to form an immunological complex containing the detection agent;
b) isolating the immunological complex from the remaining sample; and
c) determining the detection agent either in the isolated complex or in the remaining sample as an indicator of the 1-AAD-protein in the sample;
thereby determining the occurrence of AAD in the patient,
wherein the 1-AAD-protein is HMGI-C; and
wherein step a) of incubating is done at an alkaline pH.

11. The method according to claim 10, wherein the alkaline pH is pH ≥ 7.5, preferably between 8.0 and 10.0, more preferably between 8.0 and 9.5, between 8.5 and 9.5.

12. The method according to claims 10 or 11, wherein the detection agent is colloidal metallic gold.

13. A method for the immunoassay determination of acute aortic dissection (AAD) in a patient, said method comprising:
a) incubating a bodily fluid sample of the patient with
∘ at least one antibody to a first protein released during an event leading to an acute aortic dissection (1-AAD-protein) and
∘ a binding partner B for either 1-AAD-protein or the antibody
wherein either the antibody or the binding partner B is conjugated to a detection agent,
to form an immunological complex containing the detection agent;
b) isolating the immunological complex from the remaining sample; and
c) determining the detection agent either in the isolated complex or in the remaining sample as an indicator of the 1-AAD-protein in the sample;
d) comparing the said value of said 1-AAD-protein with a known reference of the respective bodily fluid of a human without acute aortic dissection;
thereby determining the occurrence of AAD in the patient,
wherein the 1-AAD-protein is HMGI-C.

14. A method for the detection of acute aortic dissection (AAD) by detecting HMGI-C in a sample of bodily fluid of a subject, the method comprising:
1) contacting the sample with a protein antibody binding to HMGI-C and thus able to form HMGI-C/Antibody-conjugates;
2) detecting the presence and quantifying the amount of said HMGI-C/Antibody-conjugate in the sample following step 1); and
3) comparing the value obtained in step 3) with a known reference.

15. The method according to claim 14, wherein the bodily fluid is blood, especially is full blood, blood plasma and/or blood serum.
